# EUROPEAN PATENT APPLICATION

(11) **EP 4 692 662 A1**
(43) Date of publication of application: **11.02.2026**
(21) Application number: 25194270.2
(22) Date of filing: 06.08.2025
(51) Int. Cl.: F24F 6/02, F24F 11/00

(54) **HUMIDIFIER**

(30) Priority: 07.08.2024 KR 20240105633
(71) Applicant: LG Electronics Inc., Yeongdeungpo-gu Seoul 07336 (KR)
(72) Inventor: JEONG, Wonjun, Seoul (KR); PARK, Keunman, Seoul (KR); KU, Myungjin, Seoul (KR); LEE, Sangheon, Seoul (KR); CHO, Kyunglok, Seoul (KR)
(74) Representative: Vossius & Partner Patentanwälte Rechtsanwälte mbB

(57) **Abstract**

A humidifier according to an embodiment of the present invention includes: a water tank forming a space in which water is held; a first humidification reservoir having a first chamber and connected to the water tank through a supply tube; a first water level sensor sensing a water level of water held within the first humidification reservoir; a heater heating water in the first chamber; a first valve opening and closing the supply tube; a second humidification reservoir having a second chamber and connected to the first humidification reservoir through a communication pipe; and a first discharge passage through which humidified air discharged from the second humidification reservoir moves, and in a passage sterilization mode, water is supplied to the first chamber up to a reference water level, and the heater is operated at a first output to boil the water within the first chamber.

## Description

The present invention relates to a humidifier, and more particularly, to a humidifier that supplies humidified clean air.

A humidifier is a device that supplies air containing moisture to a room. The humidifier includes a water tank for holding water, and is classified into an ultrasonic type, a heating type, a natural vaporization type, or a composite type according to a humidification scheme.

The humidifier is a device that vaporizes water to discharge humidified air with a high moisture content. The humidifier vaporizes water by natural vaporization, heat vaporization, and ultrasonic vibration to generate humidified air.

Each vaporization scheme has advantages and disadvantages.

In the case of the natural vaporization, there is a problem in that a user frequently has to manage a humidification medium to be used.

The ultrasonic type humidifier has a vibrator that generates vibration using ultrasonic waves, and sprays water that has been changed into a particulate form by the vibration of the vibrator, which has an advantage of a large spray amount. In the case of the vaporization by ultrasonic vibration, there is a problem in that humidified air may not move actively into an indoor space by atomizing supplied water by the ultrasonic vibration, a problem in that unpleasant humidified air may move into the indoor space when unsterilized water is humidified, and a problem in that the ultrasonic vibrator is vulnerable to high-temperature heat.

In the case of the heat vaporization, a problem of a safety accident may occur when hot humidified air is directly discharged.

Korean Patent Registration No. KR 10-0158806 as a prior document discloses a heater type ultrasonic humidifier that humidifies heated water.

In general, an air purifier and a humidifier are manufactured and used as separate products, but in response to various demands of consumers, a product having both a humidification function and an air purification function in one product is emerging. For example, Korean Patent Unexamined Publication No. 10-2005-0024044 as a prior document discloses a humidification device for air purification as well.

Meanwhile, in a humidifier or a device that provides a humidification function as in the prior documents, if water is accumulated or moisture remains in the humidifier or the device, a cleanliness state may be reduced and contaminated humidified air may be discharged.

In addition, it is necessary to manage a passage through which humidified air passes for reasons of sanitation.

The present invention has been made in view of the above problems, and it is an object of the present invention to solve the above-described problems and other problems.

An object of the present invention is to provide a humidifier capable of sterilizing a passage through which humidified air passes.

An object of the present invention is to provide a humidifier that first sterilizes a passage before a humidification operation.

An object of the present invention is to provide a humidification function and an air purification function with high reliability by checking a filter state before the humidification operation.

An object of the present invention is to provide a humidifier that generates humidified air using heated water.

An object of the present invention is to provide a humidifier with improved efficiency by seamless control logic in which passage sterilization and the humidification operation are connected.

The objects of the present invention are not limited to the aforementioned objects, and other objects, which are not mentioned above, will be apparent to a person having ordinary skill in the art from the following description.

In order to achieve the object, a humidifier according to an embodiment of the present invention may sterilize a passage through which humidified air passes during a humidification operation by allowing vapor generated by boiling water to pass through the passage.

In order to achieve the object, a humidifier according to an embodiment of the present invention effectively generates vapor and maintains a vapor generation state by controlling a water level and an output of a heater to sterilize a passage.

In order to achieve the object, a humidifier according to an embodiment of the present invention may self-check a state of a filter based on a temperature change of heated water before a humidification operation.

The invention is specified by the independent claim. Preferred embodiments are defined by the dependent claims.

A humidifier according to an embodiment of the present invention may include: a water tank forming a space in which water is held; preferably a first humidification reservoir having a first chamber and connected to the water tank through a supply tube; preferably a first water level sensor sensing a water level of water held within the first humidification reservoir; preferably a heater heating water in the first chamber; a first valve opening and closing the supply tube; preferably a second humidification reservoir having a second chamber and connected to the first humidification reservoir through a communication pipe; and preferably a first discharge passage through which humidified air discharged from the second humidification reservoir moves, and preferably in a passage sterilization mode, water may be supplied to the first chamber up to a reference water level, and the heater may be operated at a first output to boil the water within the first chamber.

An output of the heater may be switched to a second output lower than the first output, and a heating state is maintained for a predetermined time.

A sterilization operation may be repeatedly performed, which supplies water to the first chamber up to the reference water level, operates the heater at the first output, and boils the water within the first chamber, and then switches the output of the heater to the second output, and maintains the heating state for the predetermined time.

The humidifier according to an embodiment of the present invention may further include a first temperature sensor sensing a temperature of the water held within the first humidification reservoir.

When a water temperature of the first humidification reservoir reaches 100 degrees Celsius or reaches a first temperature lower than 100 degrees Celsius, and then is maintained for a reference time, this state may be determined as a boiling state.

The humidifier according to an embodiment of the present invention may further include a first connection pipe connecting the first humidification reservoir and the second humidification reservoir, in which the water heated by the first humidification reservoir moves; and a second valve opening and closing the first connection pipe.

In the passage sterilization mode, water may be supplied to the first chamber up to the reference water level, and the heater may be operated at the first output to boil the water within the first chamber, and then the first connection pipe may be opened.

The humidifier according to an embodiment of the present invention may further include a second water level sensor sensing a water level of water held within the second humidification reservoir.

When a first water level is sensed by the second water level sensor, the first connection pipe may be closed.

The humidifier according to an embodiment of the present invention may further include: a second temperature sensor sensing the temperature of the water held within the second humidification reservoir; and a filter placed below the second humidification reservoir.

After closing the first connection pipe, when a state in which the water temperature of the second humidification reservoir is equal to or higher than a preset check reference temperature is maintained for a check time or longer, a filter state check requirement notification may be output.

The humidifier according to an embodiment of the present invention may further include a blower fan placed between the first humidification reservoir and the filter, and forming air movement.

The blower fan may rotate in response to an operation of the heater.

The humidifier according to an embodiment of the present invention may further include a second discharge passage through which air filtered by the filter moves.

The humidifier according to an embodiment of the present invention may further include a mixture passage in which the air moving through the first discharge passage and the air moving through the second discharge passage are mixed.

An air volume of the air moving through the second discharge passage may be larger than an air volume of the air moving through the first discharge passage.

In the second humidification reservoir, an exhaust hole through which humidified air generated by the second chamber is discharged to the outside and an air supply hole through which air moving by the blower fan may be supplied to the second chamber.

The passage sterilization mode may be preferentially performed before a humidification operation is performed.

When water is supplied until the water level of the first humidification reservoir 300 reaches the reference water level, and the passage sterilization mode may be switched to the humidification operation.

A humidifier according to an embodiment of the present invention includes: a water tank forming a space in which water is held; preferably a first humidification reservoir having a first chamber and connected to the water tank through a supply tube; preferably a first water level sensor sensing a water level of water held within the first humidification reservoir; preferably a heater heating water in the first chamber; preferably a first valve opening and closing the supply tube; preferably a second humidification reservoir having a second chamber and connected to the first humidification reservoir through a communication pipe; preferably a first discharge passage through which humidified air discharged from the second humidification reservoir moves; preferably a first connection pipe connecting the first humidification reservoir and the second humidification reservoir, in which the water heated by the first humidification reservoir moves; and preferably a second valve opening and closing the first connection pipe, and preferably in a passage sterilization mode, water may be supplied to the first chamber up to a reference water level, and the heater may be operated at a first output to boil the water within the first chamber, and preferably the second valve opening and closing the first connection pipe may be opened to supply water to the second chamber up to a first water level.

The humidifier according to an embodiment of the present invention may further include: a first temperature sensor sensing a temperature of the water held within the first humidification reservoir; and preferably a second temperature sensor sensing a temperature of the water held within the second humidification reservoir.

The humidifier according to an embodiment of the present invention may further include a filter placed below the second humidification reservoir, and after closing the first connection pipe, when the water temperature of the second humidification reservoir does not drop, a filter state check requirement notification may be output.

When water is supplied to the first chamber up to the reference water level again, the heater may be operated at a first output, the water within the first chamber may be boiled once, and then an output of the heater may be switched to a second output lower than the first output.

After a predetermined time, water may be supplied to the first chamber up to the reference water level, the heater may be operated at the first output, the water within the first chamber may be boiled twice, and then the output of the heater may be switched to the second output.

According to at least one of the embodiments of the present invention, a passage through which humidified air passes can be sterilized, and hygiene and reliability are thus improved.

According to at least one of the embodiments of the present invention, clean humidified air may be discharged by first sterilizing the passage with vapor generated by boiling water before a humidification operation.

According to at least one of the embodiments of the present invention, by effectively determining a filter state before the humidification operation, it is possible to provide both a humidification function and an air purification function with high reliability.

According to at least one of the embodiments of the present invention, it is possible to provide clean and comfortable humidified air by generating the humidified air using heated water.

According to at least one of the embodiments of the present invention, it is possible to provide a humidifier with improved efficiency by seamless control logic in which passage sterilization and the humidification operation are connected.

The effects of the present invention are not limited to the aforementioned effect, and other effects, which are not mentioned above, will be apparent to a person having ordinary skill in the art from the description of the claims.

### BRIEF DESCRIPTION OF THE DRAWINGS

FIG. 1 is a perspective view of a humidifier according to an embodiment of the present invention.
FIG. 2 is a plan view of FIG. 1.
FIG. 3 is a cross-sectional view taken along line III-III' of FIG. 2.
FIG. 4 is an exploded perspective view of a water tank cover, an inner reservoir, an outer reservoir, an inner shell, and an outer shell according to an embodiment of the present invention.
FIG. 5 is a cross-sectional view of the inner reservoir according to an embodiment of the present invention.
FIG. 6 is an exploded perspective view of a water tank, the inner shell, and a middle tray according to an embodiment of the present invention.
FIG. 7 is a perspective view of a middle tray according to an embodiment of the present invention.
FIG. 8 is a cross-sectional perspective view of one side in which a structure including the water tank, the inner shell, and the middle tray is coupled according to an embodiment of the present invention.
FIG. 9 is a side view of a structure in which a first humidification reservoir and a second humidification reservoir are coupled according to an embodiment of the present invention.
FIG. 10 is a side view in another direction of the structure in which the first humidification reservoir and the second humidification reservoir are coupled according to an embodiment of the present invention.
FIG. 11 is a perspective view for describing top surfaces of the first humidification reservoir and the second humidification reservoir according to an embodiment of the present invention.
FIG. 12 is a cross-sectional view in one direction of the structure in which the first humidification reservoir and the second humidification reservoir are coupled according to an embodiment of the present invention.
FIG. 13 is a cross-sectional view in a direction different from that of FIG. 12.
FIG. 14 is a schematic diagram for describing a specific connection relationship between the first humidification reservoir and the second humidification reservoir, and a placement relationship of internal components according to an embodiment of the present invention.
FIG. 15 is a block diagram of a humidifier according to an embodiment of the present invention.
FIG. 16 is a flowchart illustrating a method for operating a humidifier according to an embodiment of the present invention.
FIGS. 17A to 17I are diagrams referred to in the description of a passage sterilization mode according to an embodiment of the present invention.
FIG. 18 is a diagram referred to in the description of passage sterilization mode setting of the humidifier according to an embodiment of the present invention.
FIG. 19 is a flowchart illustrating a method for operating a humidifier according to an embodiment of the present invention.
FIG. 20 is a flowchart illustrating a method for operating a humidifier according to an embodiment of the present invention.

### DETAILED DESCRIPTION OF THE PREFERRED EMBODIMENTS

Hereinafter, embodiments disclosed in the present invention will be described in detail with reference to the accompanying drawings. Like reference numerals refer to like or similar elements regardless of reference numerals and a duplicated description thereof will be omitted.

Suffixes "module" and "unit" for components used in the following description are given or mixed in consideration of easy preparation of the present invention only and do not have their own distinguished meanings or roles.

Terms including an ordinary number, such as first, second, etc., are used for describing various components, but the components are not limited by the terms. The terms are used only to determine one component from another component.

It should be understood that, when it is described that a constituent element is "connected to" or "accesses" another constituent element, the constituent element may be directly connected to or access the other constituent element, or a third constituent element may be present therebetween. In contrast, when it is described that a constituent element is "directly connected to" or "directly accesses" another constituent element, it is understood that no constituent element is present between the constituent element and another constituent element.

A singular form may include a plural form unless otherwise clearly defined in the context.

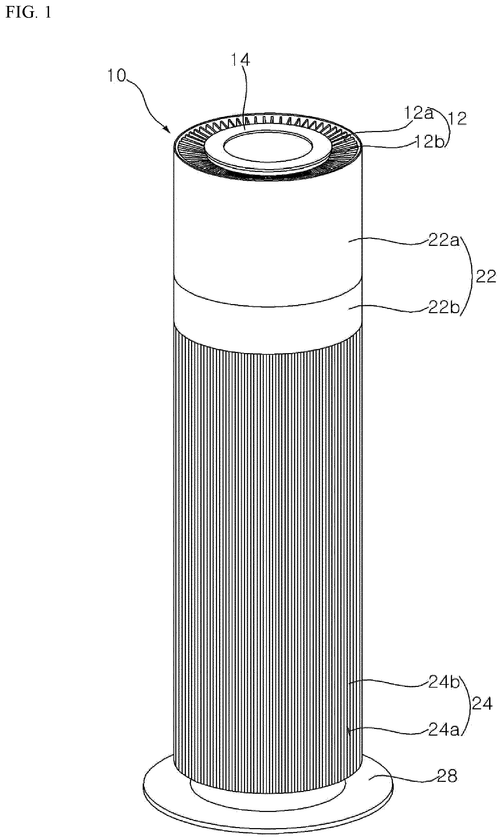

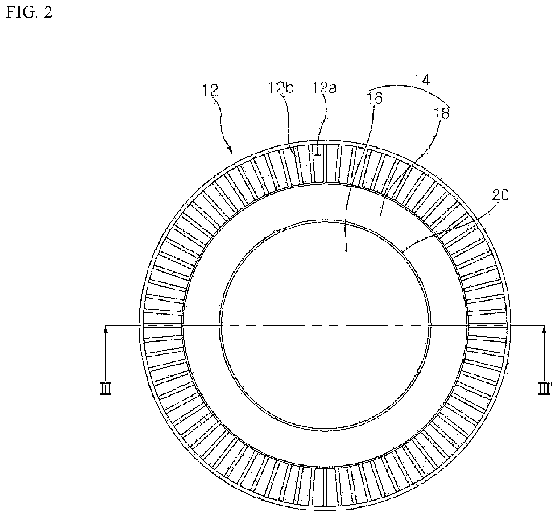

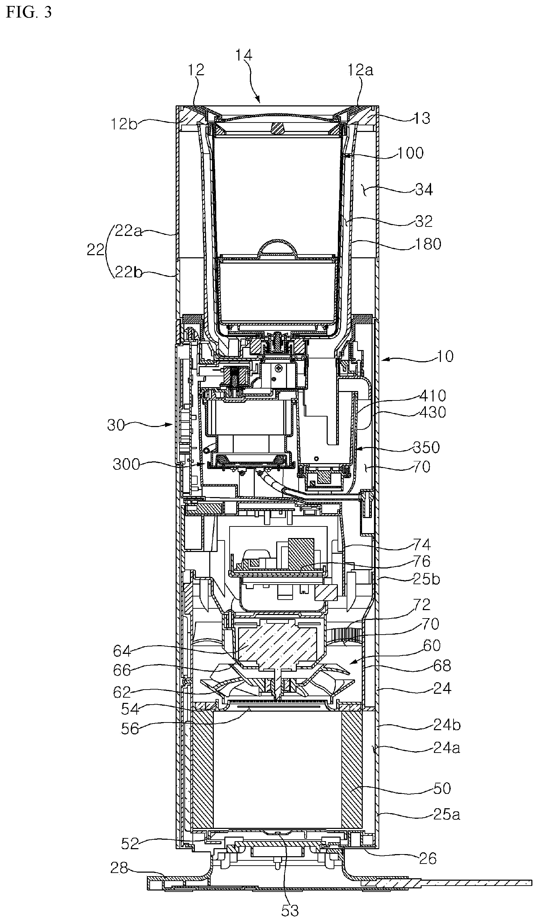

FIG. 1 is a perspective view of a humidifier according to an embodiment of the present invention. FIG. 2 is a plan view of FIG. 1 and FIG. 3 is a cross-sectional view taken along line III-III' of FIG. 2.

First, an overall configuration of a humidifier according to the present invention will be described with reference to FIGS. 1 to 3.

The humidifier of the present invention may humidity water by ultrasonic vibration. The humidifier of the present invention may discharge humidified air by heating water. The humidifier of the present invention may discharge the humidified air generated by the ultrasonic vibration and the humidified air generated by heating.

Referring to FIGS. 1 to 3, a humidifier may include a casing 10 that forms an exterior, with an inlet 24a and an outlet 12a formed therein, a filter unit that is placed inside the casing 10, and filters air admitted into the inlet 24a, a blower device 60 that is placed inside the casing 10, and moves some of the air inside the casing 10 from the inlet 24a to the outlet 12a, and a humidification module that is placed inside the casing 10, and humidifies some of the air moving to the blower device 60.

The humidification module may include a first humidification reservoir 300 that heats water and a second humidification reservoir 350 that generates humidified air with water. The humidification module may include a humidification module housing 410 that covers peripheries of the first humidification reservoir 300 and the second humidification reservoir 350. A channel housing 430 may be placed on an outer periphery of the humidification module housing 410. A blower channel 70 may be formed between the humidification module housing 410 and the channel housing 430. A configuration and a placement of the humidification module will be described in detail below.

The casing 10 may have a generally cylindrical shape.

The casing 10 may include an intake grille 24 that forms the inlet 24a through which air is admitted, and a discharge grille 12 that forms the outlet 12a through which air is discharged.

The inlet 24a may be formed on a peripheral surface of the casing 10 having the cylindrical shape. The outlet 12a may be formed on a top surface of the casing 10 having the cylindrical shape. In the humidifier of the present invention, air may be admitted through the circumferential surface and air may be discharged through the top surface.

Referring to FIG. 3, the intake grille 24 may cover outsides of a filter 50 and a blowing housing 68 to be described below. A plurality of inlets 24a may be formed in a vertical direction and spaced apart from each other in a circumferential direction may be formed in the intake grilles 24. The inlet 24a may be formed around a location where the filter 50 is placed. The inlet 24a may be formed in a lower portion of the intake grille 24. The lower portion of the intake grille 24 may be formed with inlets 24, and an upper portion of the intake grille 24 may be enclosed to protect the internal construction of the humidifier.

A plurality of grilles 24b which extend in the vertical direction may be placed in the intake grilles 24. The plurality of grilles 24b may be placed to be spaced in the circumferential direction of the intake grille 24. A plurality of inlets 24a may be formed between the plurality of grilles 24b.

The intake grille 24 may be divided into an intake grille lower portion 25a in which the inlet 24a is formed and an intake grille upper portion 25b placed above the intake grille lower portion 25a. A display 30 to be described below may be placed in the intake grille upper portion 25b. The intake grille upper portion 25b may cover the blowing housing 68 and an outside of the humidification module to be described below.

The humidifier may include the discharge grille 12 that forms the outlet 12a and a water tank cover 14 which is placed on a top side of the water tank 100. The discharge grille 12 may have a structure that is separated upward from an outer shell 22 described below. The water tank cover 14 may have a structure that is separated from the discharge grille 12 or the water tank 100. The discharge grille 12 may include a plurality of ribs 12b extending radially on an outer periphery of the water tank cover 14. The plurality of ribs 12b placed in the discharge grille 12 may be placed to be spaced in the circumferential direction on the outer periphery of the water tank cover 14.

Referring to FIG. 2, the water tank cover 14 may include a central cover 16 and a peripheral cover 18 placed on the periphery of the central cover 16. The central cover 16 may have a structure that bulges upward toward the center. A water supply hole 20 may be formed between the peripheral cover 18 and the central cover 16 to allow water to move into the water tank 100.

Referring to FIG. 1, the discharge grille 12 may be placed above a first discharge passage 32 and above a second discharge passage 34 to be described below. A plurality of ribs 12b and a plurality of outlets 12a disposed in the discharge grille 12 may be formed.

The discharge grille 12 may have a constant height in the vertical direction. Thus, a mixture passage 13 may be formed between the plurality of ribs 12b placed in the discharge grille 12. In the mixture passage 13, the air moving through the first discharge passage 12 and the air moving through the second discharge passage 34 may be mixed together.

Each of the plurality of ribs 12b may be formed in such a way that an outer peripheral end thereof is positioned higher than an inner peripheral end thereof. Thus, the air moving through the first discharge passage 32 and the second discharge passage 34 may be guided radially inwardly.

The casing 10 may include an outer shell 22 that guides air moving therein to the outlet 12a. The outer shell 22 may form the second discharge passage 34 with the inner shell 180 to be described below.

The outer shell 22 may include an upper outer shell 22a placed under the discharge grille 12 and a lower outer shell 22b placed under the upper outer shell 22a. The lower outer shell 22b may be made of a transparent material.

The casing 10 may include a lower wall 26 that covers a bottom side of the intake grille 24.

The humidifier may include a base 28 placed on a bottom side of the casing 10, and causing a bottom wall 26 to be spaced upward from the ground. An upper end portion of the base 28 may be connected to the bottom wall 26. The bottom wall 26 may be placed to cover a bottom surface of the humidifier that is spaced upwardly from the ground by the base 28.

Referring to FIG. 2, the display 30 is placed on one side of the casing 10. The display 30 may allow a user to control a power or an operation of the humidifier. A display for showing an operation status of the humidifier to the user may be placed in the display 30.

The filter unit may allow the air admitted through the inlet 24a to be filtered through the filter 50. The filter unit may allow the filtered air to move upward.

Referring to FIG. 3, the filter unit may include a filter 50 for filtering the air admitted through the inlets 24a and a filter mounting portion for fixing the filter 50 in place inside the casing 10.

The filter 50 may have the cylindrical shape. Thus, the filter 50 may be able to filter air sucked from longitudinal and lateral directions perpendicular to the vertical direction. The air admitted through the inlet 24a may move to an inside space of the filter 50. The air that passes through the filter 50 may move to the blower device 60 placed above the filter 50.

The filter mounting portion may include a lower plate 52 placed on a lower side of the filter 50, an upper plate 54 placed on a top side of the filter 50, and a supporter (not illustrated) connecting the lower plate 52 and the upper plate 54.

The lower plate 52 may be placed on the lower side of the filter 50. The lower plate 52 may move up and down, and detect whether the filter 50 is placed. A fan sterilizer 53 that emits ultraviolet light upward may be placed in the center of the lower plate 52. The fan sterilizer may sterilize a blower fan 62 to be described below or the inside of the filter 50.

An orifice 56 may be formed on the upper plate 54. The orifice 56 may be formed in the center of the upper plate 54. The orifice 56 may allow the air admitted to the inside of the filter 50 to move to the blower fan 62. An inner circumferential end of the upper plate 54 may be bent upward so as to guide air moving upward in an internal space to the blower fan 62.

The supporter may connect the lower plate 52 and the upper plate 54. The supporter may be placed to be spaced in the circumferential direction.

The blower 60 may include the blower fan 62 that forms an air movement within the casing 10 and a fan motor 64 that rotates the blower fan 62.

Referring to FIG. 3, the blower fan 62 may have a fan intake opening on one side facing the orifice 56 and a fan discharge opening on an opposite side to the fan intake opening. As the blower fan 62, a diagonal flow fan in which the fan discharge opening on the opposite side of the fan intake opening faces in a centrifugal direction may be used. The blower fan 62 may include a hub connected to the fan motor 64, a shroud placed to be spaced apart from the hub by a certain distance and forming the fan intake opening, and a blade extending radially connect to connect the hub and the shroud.

The blower fan 62 may operate to send air upward from below. The blower fan 62 may suck air toward the orifice 56 and discharge air to the blower housing 68 where a diffuser 72 is placed.

The fan motor 64 may be placed above the blower fan 62.

The blower may include a motor cover 66 that covers the outside of the fan motor 64, and a blower housing 68 placed to be spaced radially outward from the motor cover 66, that guides the air moving by the blower fan 62 to upward.

A blower channel 70 along which the air moving by the blower fan 62 moves upward may be formed between the motor cover 66 and the blower housing 68. The blower channel 70 may extend to a region where a humidification module housing 410 and a channel housing 430 are formed.

The intake grille 24 may be placed on the outside of the blower housing 68.

The blower 60 may include a diffuser 72 placed between the motor cover 66 and the blower housing 68, which reduces rotational components of the air moving upward by the blower fan 62. A plurality of diffusers 72 may be spaced apart from each other in the circumferential direction.

A control box 74 forming a space in which a circuit board 76 is placed may be placed above the motor cover 66. The control box 74 may be spaced apart inwardly from the blower housing 68. Thus, the blower channel 70 may also be formed in the space between the control box 74 and the blower housing 68.

One or more circuit boards 76 may be placed within the control box 74.

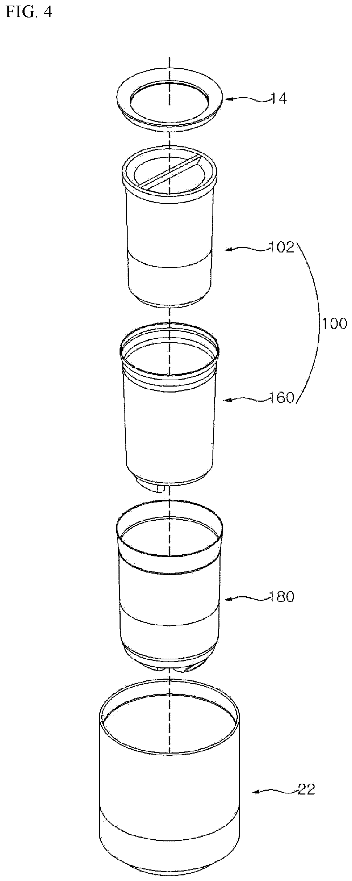

FIG. 4 is an exploded perspective view of a water tank cover, an inner reservoir, an outer reservoir, an inner shell, and an outer shell according to an embodiment of the present invention.

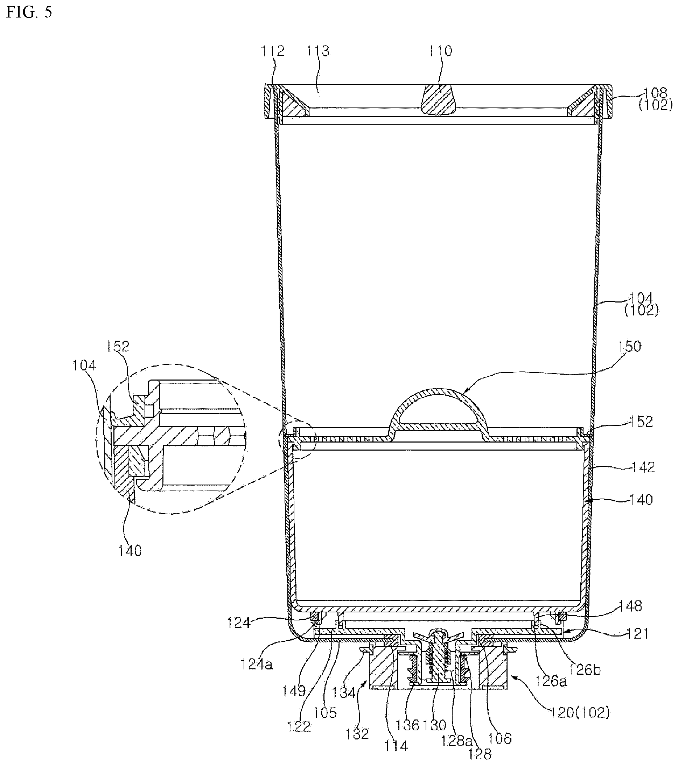

FIG. 5 is a cross-sectional view of the inner reservoir according to an embodiment of the present invention.

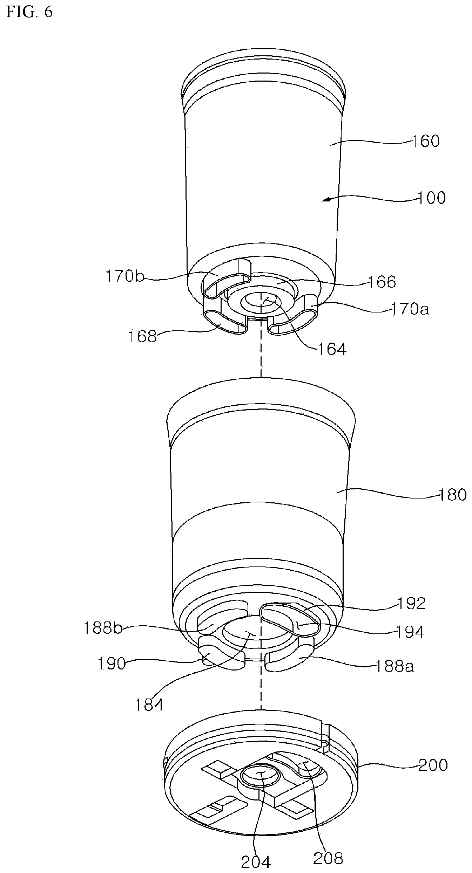

FIG. 6 is an exploded perspective view of a water tank, the inner shell, and a middle tray according to an embodiment of the present invention.

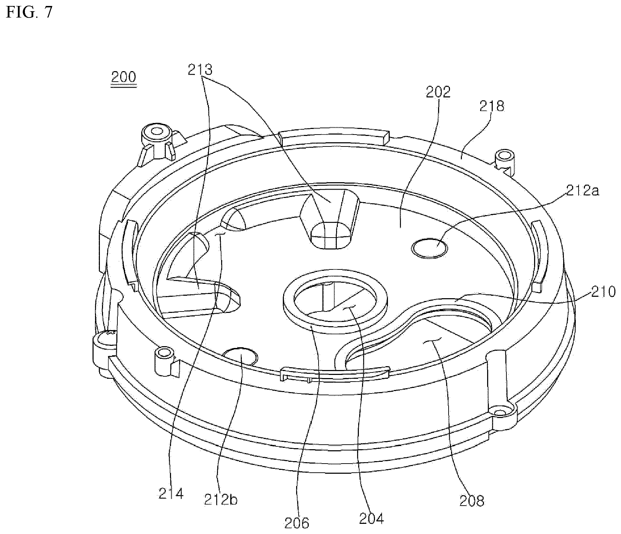

FIG. 7 is a perspective view of a middle tray according to an embodiment of the present invention.

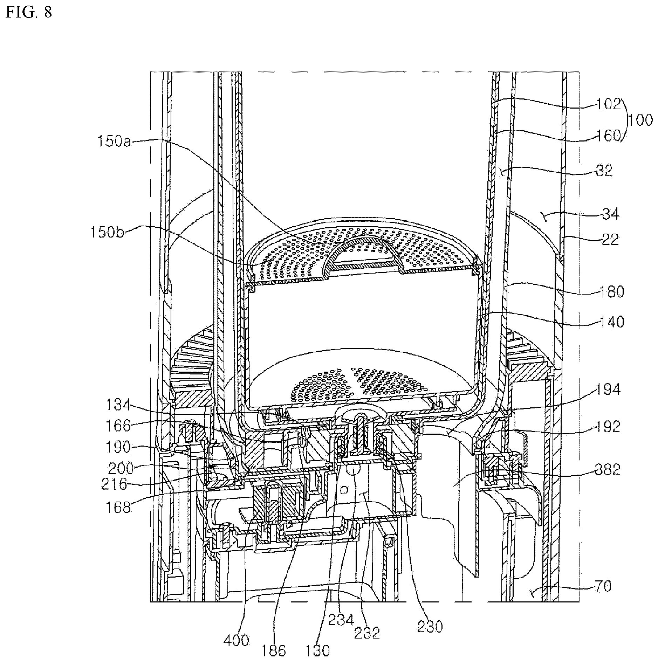

FIG. 8 is a cross-sectional perspective view of one side in which a structure including the water tank, the inner shell, and the middle tray is coupled according to an embodiment of the present invention.

Hereinafter, with reference to FIGS. 4 to 8, a construction in which the discharge passage of the present invention is formed and a construction of the water tank 100 holding water will be described.

The humidifier may include a water tank 100 that forms a space in which water is held, an inner shell 180 that forms a space in which the water tank 100 is placed, and an outer shell 22 that is spaced apart from an outer periphery of the inner shell 180. The humidifier may include a water softening device 14 placed on the top of the water tank 100.

The water tank 100 may include an inner reservoir 102 in which water is held and an outer reservoir 160 placed to cover the outside of the inner reservoir 102. The inner reservoir 102 may be placed inside the outer reservoir 160. When the inner reservoir 102 is placed inside the outer reservoir 160, an outer peripheral surface of the inner reservoir 102 may be placed in contact with an inner peripheral surface of the outer reservoir 160.

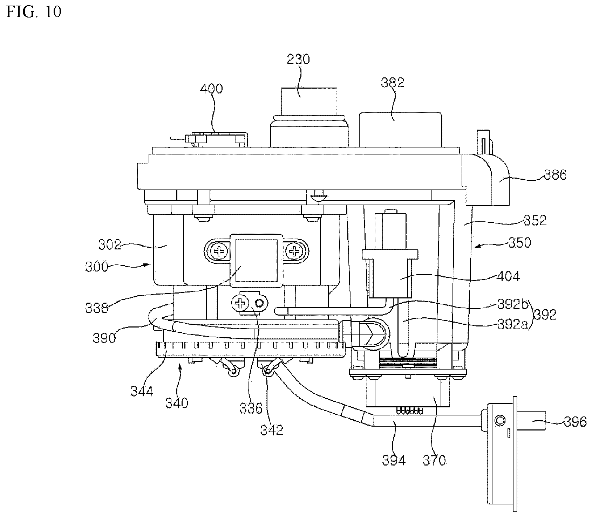

Referring to FIG. 5, the inner reservoir 102 may include an inner reservoir 104 that forms a space in which water is held, a handle 108 that is placed on a top side of the inner reservoir 102, and a connector 120 placed on a bottom side, which connects the inner reservoir 104 to a supply tube 230 (see FIG. 10) to be described below.

Referring to FIG. 5, a water softener 140 that softens water in the inner reservoir 102 may be placed within the inner reservoir 102. The water softer 140 may include a water softener housing 142 forming a space in which an ion exchange resin filter (not illustrated) is placed therein, and a housing cover 150 that covers a top side of the water softener housing 142.

A plurality of cover holes 150b may be formed in the housing cover 150, and a plurality of housing holes (not illustrated) may be formed in the water softener housing 142.

The inner reservoir 104 may be formed in an approximately cylindrical shape. The inner reservoir 104 may have a shape in which a top side is opened. A bottom hole 106 through which held water is discharged may be formed on a bottom surface of the inner reservoir 104. Referring to FIG. 5, the inner reservoir 104 may have a shape in which an internal diameter of a reservoir decreases from the top side to the bottom side.

The bottom hole 106 may be formed at a center of the bottom surface of the inner reservoir 104. The connector 120 may be fixedly placed in the bottom hole 106. The water held in the inner reservoir 102 may move to the supply tube 230 through the connector 120 mounted on the bottom hole 106.

The water softener 140 may be fixedly placed within the inner reservoir 102. The water softener 140 may have an ion exchange resin (not illustrated) that removes ions from water admitted into the inner reservoir 102 therein. The water softener 140 may include a gasket 152 that seals an inner peripheral surface of the inner reservoir 102 and an outer peripheral surface of the water softener 140 on one side of a circumferential surface thereof.

Referring to an enlarged diagram, the gasket 152 placed at an upper end portion of the water softener 140 may be placed in contact with an inner circumferential surface of the inner reservoir 102. Thus, water is prevented from moving to a space between the inner reservoir 102 and the water softener 140 without passing through the inside of the water softener 140. Water admitted from the top side of the inner reservoir 102 and moving to the bottom hole 106 of the inner reservoir 102 may pass through the water softener 140.

The handle 108 may include a ring-shaped handle fixing portion 112 fixedly placed on the upper end portion of the inner reservoir 104 and a handle bar 110 placed to cross the top side of the inner reservoir 104.

The handle fixing portion 112 may have a structure that is hooked to an upper end of the inner reservoir 104. A region of the handle fixing portion 112 placed inside the inner reservoir 104 may include an inclination wall 113 that is inclined downward with respect to the inside of the inner reservoir 104. Accordingly, water dropped through the water tank cover 14 may be admitted into the inner reservoir 102 along an inclination surface of the handle fixing portion 112.

Referring to FIG. 5, the connector 120 may include a connector body 121 placed so as to pass through the bottom hole 106 of the inner reservoir 104, a connector holder 132 coupled to the connector body 121 to fix placement of the connector body 121, and a connector valve 130 placed inside the connector body 121, which opens and closes an internal passage of the connector body 121.

Referring to FIG. 5, the connector body 121 may include a connector plate 122 placed inside the inner reservoir 104, and a connector pipe 128 placed to pass through the bottom hole 106 of the inner reservoir 104 and having an internal passage 128a therein. The connector plate 122 may be placed on the top side of the inner reservoir 104.

The connector plate 122 may have a structure that is in contact with a bottom of the water softener 140 to fix the placement of the water softener 140. Fixing ribs 126a and 126b that protrude upward may be placed in the connector plate 122.

Referring to FIG. 5, the fixing ribs 126a and 126b may include a first fixing rib 126a, and a second fixing rib 127b placed radially spaced apart from the first fixing rib 126a. A lower projection 148 of the water softener 140 may be placed in a space between the first fixing rib 126a and the second fixing rib 126b. The first fixing rib 126a and the second fixing rib 126b may prevent the water softener 140 placed inside the inner reservoir 102 from moving in a direction perpendicular to the vertical direction.

A rim wall 124 placed more outward radially outward of the fixing ribs 126a and 126b and protruding upward may be placed on the connector plate 122. Referring to FIG. 5, a hook hole 124a into which a hook 149 of the water softener 140 is inserted may be formed in the rim wall 124.

The connector pipe 128 may have a structure that extends downward from the center of the connector plate 122. The connector pipe 128 may be placed to pass through the bottom hole 106 of the inner reservoir 104. The internal passage 128a through which water inside the inner reservoir 102 moves may be formed inside the connector pipe 128. A connector valve 130 that opens and closes the internal passage 128a may be placed inside the connector pipe 128.

The connector valve 130 may be placed to move vertically inside the connector pipe 128. When the connector valve 130 is connected to the supply tube 230 described below, the connector valve 130 may move upward to open the internal passage 128a.

The connector holder 132 may be fixedly placed on an outer periphery of the connector pipe 128. The connector holder 132 may be placed below the bottom surface of the inner reservoir 104. Thus, the connector holder 132 may be connected to the connector pipe 128 to fix the placement of the connector body 121.

Referring to FIG. 5, the inner reservoir 102 may include a packing 114 mounted to the inner reservoir 104 that forms a periphery of the bottom hole 106. The packing 114 may be made of a rubber material. The packing 114 may be placed to be in contact with the connector plate 122 upward and to be in contact with the connector holder 132 downward.

The packing 114 may prevent a contact between the inner reservoir 104 and the connector body 121. The packing 114 may prevent a contact between the inner reservoir 104 and the connector holder 132. The packing 114 may seal a gap between the connector plate 122, and the bottom wall 105 of the inner reservoir 104. Further, the packing 114 may seal a gap between the connector holder 132, and the bottom wall 105 of the inner reservoir 104. That is, the packing 114 may double prevent water present inside the inner reservoir 102 from leaking through the outside of the connector 120.

An inner sealer 136 may be placed on an outer periphery of the connector pipe 128. The inner sealer 136 may seal a gap between the connector 120 and the supply tube 230.

An outer sealer 134 may be placed on an outer periphery of the connector holder 132. The outer sealer 134 may be placed to be in contact with one side of the outer reservoir 160 and/or the inner shell 180 to be described below.

Referring to FIG. 4, the water tank cover 14 may be placed above the inner reservoir 102. The water tank cover 14 may be placed to be seated on the discharge grille 12. The water tank cover 14 may include a central cover 16, and a peripheral cover 18 placed on an outer periphery of the central cover 16.

The peripheral cover 18 may be placed above the discharge grille 12 to maintain the placement of the water tank cover 14. The peripheral cover 18 may have a ring-shaped structure. The peripheral cover 18 may have a structure that is inclined downward toward a radial inner side. Therefore, when water is supplied to the top, water may be moved to the central cover 16 side.

The central cover 16 may have a shape that bulges upward. Therefore, when water is supplied to the top side of the central cover 16, the water may move in an outer peripheral direction of the central cover 16. A water supply hole 20 for supplying water toward the inner reservoir 102 may be formed between the central cover 16 and the peripheral cover 18. The water supply hole 20 may move above the handle fixing portion 112 of the handle 108 of the inner reservoir 102.

The water tank cover 14 may be placed to be spaced apart upward from the inner reservoir 102 and/or the outer reservoir 160. Therefore, a force of pressing the water tank cover 14 downward by water in the process of supplying water may not be transmitted to the inner reservoir 102 or the outer reservoir 160.

The outer reservoir 160 may be placed on an outer periphery of the inner reservoir 102. The outer reservoir 160 may be placed to be in contact with the outer periphery of the inner reservoir 102. Thus, it is possible to minimize condensate water generated in the outer periphery of the inner reservoir 102.

An upper end portion 162 of the outer reservoir 160 may be formed to be spaced apart from the inner reservoir 102. That is, the upper end portion 162 of the outer reservoir 160 may be formed such that a ratio of radially expanding toward the top side increases.

The outer reservoir 160 may also have a cylindrical shape in which a top side is opened. A through-hole 164 through which the connector 120 of the inner reservoir 102 passes may be formed on a bottom surface of the outer reservoir 160. The through-hole 164 may be formed to be larger than the bottom hole 106 of the inner reservoir 102.

Referring to FIG. 6, the bottom surface of the outer reservoir 160 may be placed with a lower projection 166 protruding downward from a periphery of the through-hole 164, a pair of first water tank projections 170a and 170b protruding downward from the bottom surface of the outer reservoir 160, and a second water tank projection 168 protruding downward from the bottom surface of the outer reservoir 160.

The lower projection 166 may be placed on the periphery of the through-hole 164. The lower projection 166 may extend downward on an inner circumferential end of the bottom surface of the outer reservoir 160. The lower projection 166 may have a ring-shaped structure. The outer sealer 134 may be placed in contact with an inner circumferential surface of the lower projection 166.

Referring to FIG. 6, the pair of first water projections 170a and 170b may be placed in opposite directions to each other with respect to the through-hole 164. The pair of first water tank projections 170a and 170b may transmit loads of the inner reservoir 102 and the outer reservoir 160 to weight sensors 212a and 212b of the middle tray 200 to be described below. The pair of first water tank projections 170a and 170b may transmit the loads of the inner reservoir 102 and the outer reservoir 160 directly to the weight sensors 212a and 212b or transmit the loads indirectly through the inner shell 180.

The second water tank projection 168 may be placed between the pair of first water tank projections 170a and 170b.

The inner shell 180 may be placed to be spaced apart from the outer reservoir 160. The inner shell 180 may be placed to be spaced apart from the water tank 100. A first discharge passage 32 through which humidified air moves may be formed between the inner shell 180 and the outer reservoir 160.

Referring to FIG. 8, the inner shell 180 may be placed to be spaced apart from the outer shell 22. A second discharge passage 34 through which filtered air moves may be formed between the inner shell 180 and the outer shell 22.

The inner shell 180 may have a cylindrical shape in which a top side is opened. The inner shell 180 may have a larger diameter than the outer reservoir 160. A ratio at which an upper end portion of the inner shell 180 is expanded radially may be formed to be large like the upper end portion 162 of the outer reservoir 162.

A shell through-hole 184 may be formed on a bottom surface of the inner shell 180. The shell through-hole 184 may be placed below the through-hole 164 of the outer reservoir 160. The connector 120 may be placed in the shell through-hole 184.

The inner shell 180 may be made of a transparent material. Referring to FIG. 8, an upper projection 186 protruding upward may be placed in a portion where the shell through-hole 184 is formed. The upper projection 186 of the inner shell 180 may be placed to be in contact with the lower projection 166 of the outer reservoir 160.

A size of the shell through-hole 184 of the inner shell 180 may be formed to be smaller than a size of the through-hole 164 of the outer reservoir 160. The upper projection 186 may be placed inside the lower projection 166 of the outer reservoir 160. That is, an outer circumferential surface of the upper projection 186 may be placed to face an inner circumferential surface of the lower projection 166 of the outer reservoir 160.

The outer sealer 134 may be placed above the upper projection 186.

In the inner shell 180, a pair of first shell projections 188a and 188b on which the pair of first water tank projections 170a and 170b of the outer reservoir 160 are mounted may be placed. The pair of first shell projections 188a and 188b may have a structure protruding downward from the bottom surface of the inner shell 180. Spaces into which the pair of first water tank projections 170a and 170b are inserted are formed in the pair of first shell projections 188a and 188b, respectively.

The pair of first shell projections 188a and 188b may be placed above the weight sensors 212a and 212b placed in the middle tray 200. Thus, the pair of first shell projections 188a and 188b may transmit the loads of the inner reservoir 102 and the outer reservoir 160 to the weight sensors 212a and 212b.

A second shell projection 190 on which the second water tank projection 168 of the outer reservoir 160 is mounted may be placed in the inner shell 180. A space into which the second water tank projection 168 is inserted may be formed in the second shell projection 190.

Referring to FIG. 8, an exhaust connection pipe 192 that guides humidified air discharged from the second humidification reservoir 350 to the first discharge passage 32 may be placed in the inner shell 180. The exhaust connection pipe 192 may have a structure extending downward on the bottom surface of the inner shell 180. The exhaust connection pipe 192 may have a shape corresponding to an exhaust pipe 382 to be described below. The exhaust connection pipe 192 may have an approximately elliptical shape.

Referring to FIG. 8, a lower end portion of the exhaust connection pipe 192 may be connected to an upper end portion of the exhaust pipe 382. The lower end portion of the exhaust connection pipe 192 may be placed inward from the upper end portion of the exhaust pipe 382. The lower end portion of the exhaust connection pipe 192 may be formed with a step for protruding inward of the exhaust pipe 382. The lower end portion of the exhaust connection pipe 192 may be formed with an exhaust pipe connection hole 194 opened downward.

The humidifier may include a middle tray 200 that partitions a region in which the water tank 100 is placed and a region in which the humidification module is placed. The middle tray 200 may sense a water level of water held in the water tank 100. The middle tray 200 may emit light to discharged humidified air.

Referring to FIG. 6, the middle tray 200 may be placed below the water tank 100. The middle tray 200 may be placed above the second humidification reservoir 350 or the first humidification reservoir 300 to be described below. Weight sensors 212a and 212b that sense the water level of the water tank 100 by a weight may be placed in the middle tray 200.

Referring to FIG. 8, a lamp 216 that emits light toward the first discharge passage 32 may be placed in the middle tray 200. The inner shell 180 may be seated on the middle tray 200. The inner reservoir 102 and the outer reservoir 160 may be mounted on the middle tray 200.

Referring to FIG. 7, the middle tray 200 may include a tray plate 202, and a lamp housing 218 placed on an outer periphery of the tray plate 202 and having the lamp 216 placed therein.

A first hole 204 through which the supply tube 230 passes may be formed on one side of the tray plate 202. The first hole 204 may be formed at the center of the tray plate 202. A part of the supply tube 230 is placed above the first hole 204. The connector 120 of the inner reservoir 102 may be connected to the supply tube 230 placed at a top side of the first hole 204.

Referring to FIG. 7, a first flange 206 protruding upward of the tray plate 202 may be placed on a periphery of the first hole 204. Thus, water admitted through a top side of the tray plate 202 may be prevented from moving downward of the middle tray 200.

A second hole 208 through which humidified air discharged from the second humidification reservoir 350 moves may be formed on the other side of the tray plate 202. A second flange 210 protruding upward of the tray plate 202 may be placed on a periphery of the second hole 204. The second flange 210 may prevent the water admitted through the top side of the tray plate 202 from moving downward of the middle tray 200 through the second hole 208.

The lamp housing 218 may be placed along the periphery of the tray plate 202. The lamp housing 218 may be formed in a ring shape. A lamp 216 that emits light upward may be placed within the lamp housing 218. One side of the lamp housing 218 may be made of a material that transmits the light emitted from the lamp 216.

The middle tray 200 may be placed to cover the top sides of the first humidification reservoir 300 and the second humidification reservoir 350. The middle tray 200 may be coupled to a humidification module housing 410 to be described below to form a space in which the first humidification reservoir 300 and the second humidification reservoir 350 are placed.

The middle tray 200 may be placed on top sides of the first humidification reservoir 300 and the second humidification reservoir 350. The middle tray 200 may be placed on an opened top side of the humidification module housing 410. The middle tray 200 may cover the opened top side of the humidification module housing 410.

The water tank 100 may be mounted on the top side of the middle tray 200. The water tank 100 and the inner shell 180 may be placed on the top side of the middle tray 200. Referring to FIG. 7, weight sensors 212a and 212b that sense the water level of the water tank 100 may be placed on a top surface of the middle tray 200.

A second hole 208 formed so that the humidified air discharged from the second humidification reservoir 350 moves may be formed on one side of the top surface of the middle tray 200. A partial configuration of the supply tube 230 may protrude above the middle tray 200 through the first hole 204 formed in the middle tray 200.

A mounting projection 213 protruding upward may be placed on the top surface of the middle tray 200 so as to maintain the placement of the second shell projection 190 of the inner shell 180. The mounting projection 213 may be placed on a periphery of a fixing groove 214 in which the second shell projection 190 is placed.

A peripheral wall 327 of a second top cover 326 to be described below may be placed to be exposed between the middle tray 200 and the humidification module housing 410.

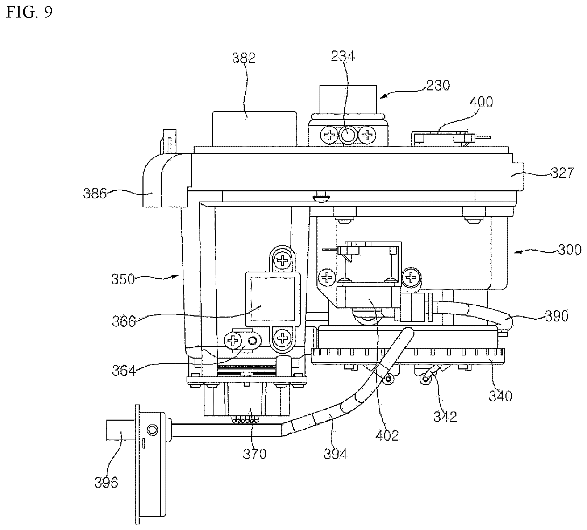

FIG. 9 is a side view of a structure in which a first humidification reservoir and a second humidification reservoir are coupled according to an embodiment of the present invention and FIG. 10 is a side view in another direction of the structure in which the first humidification reservoir and the second humidification reservoir may be coupled according to an embodiment of the present invention.

Referring to FIGS. 9 and 10, a drain pipe valve 396 for opening and closing a drain pipe 394 may be placed at an end portion of the drain pipe 384. The drain pipe valve 396 may open and close the drain pipe 394 depending on the placement.

The display 30 may be mounted on the other side of the peripheral surface of the humidification module housing 410.

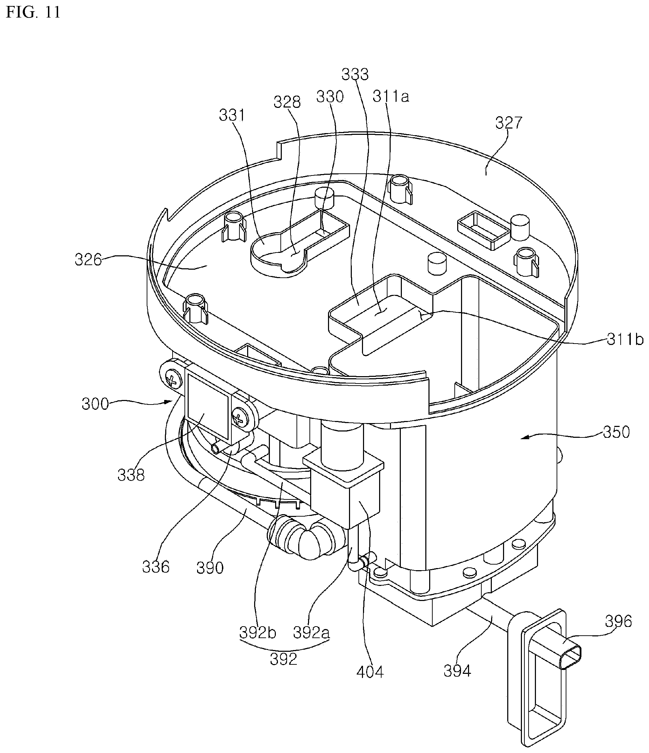

FIG. 11 is a perspective view for describing top surfaces of the first humidification reservoir and the second humidification reservoir according to an embodiment of the present invention.

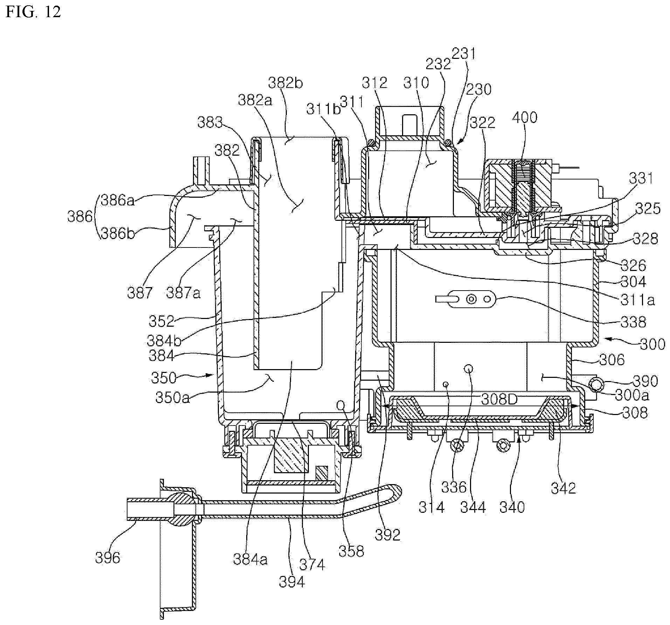

FIG. 12 is a cross-sectional view in one direction of the structure in which the first humidification reservoir and the second humidification reservoir are coupled according to an embodiment of the present invention.

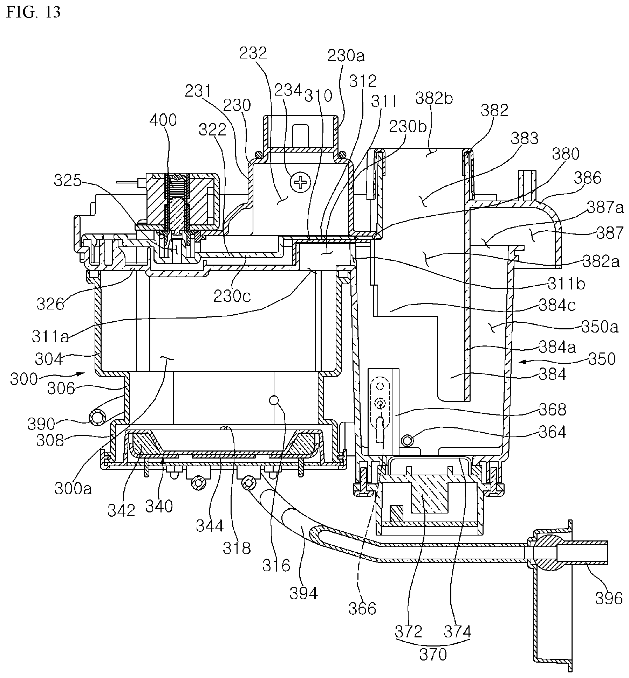

FIG. 13 is a cross-sectional view in a direction different from that of FIG. 12.

Constructions of the supply tube, the first humidification reservoir, the second humidification reservoir, and the exhaust pipe will be described with reference to FIGS. 9 to 13.

The humidifier of the present invention may include a humidification module that heats water and generates humidified air. The humidification module may include a first humidification reservoir 300 that heats water and a second humidification reservoir 350 that generates humidified air with a vibrator 370.

The humidifier may include a first connection pipe 390 that connect the first humidification reservoir 300 and the second humidification reservoir 350, and a second connection pipe 392. The first connection pipe 390 may supply water heated by the first humidification reservoir 300 to the second humidification reservoir 350. Referring to FIG. 9, a second valve 402 may be placed in the first connection pipe 390. The second valve 402 may open and close an internal passage of the first connection pipe 390 to supply the water heated by the first humidification reservoir 300 to the second humidification reservoir 350.

The first humidification reservoir 300 and the second humidification reservoir 350 may be connected through a communication pipe 310 placed above the first connection pipe 390.

The water heated by the first humidification reservoir 300 may be supplied to the second humidification reservoir 350 through the first connection pipe 390. Humidified air generated by the first humidification reservoir 300 may move to the second humidification reservoir 350 through the communication pipe 310.

Referring to FIG. 10, a pump 404 may be placed in the second connection pipe 392. When the pump 404 operates, the water in the second humidification reservoir 350 may move to the first humidification reservoir 300.

The humidifier may include a first temperature sensor 336 that senses a temperature of the water held within the first humidification reservoir 300, and a first water level sensor 338 that senses a water level of the water held within the first humidification reservoir 300. Referring to FIG. 10, the first temperature sensor 336 and the first water level sensor 338 may be placed on one side of the first humidification reservoir 300.

The humidifier may include a second temperature sensor 364 that senses a temperature of the water held within the second humidification reservoir 350, and a second water level sensor 366 that senses a water level of the water held within the second humidification reservoir 350. Referring to FIG. 9, the second temperature sensor 364 and the second water level sensor 366 may be placed on one side of the second humidification reservoir 350.

The humidifier may include a sensor 234 that senses a water quality of water present within the supply tube 230. Referring to FIG. 9, the sensor 234 may be placed on one side of the supply tube 230. The sensor 234 may sense a water quality of water moving from the water tank 100 The sensor 234 may sense the water quality of water to sense a replacement time of the water softener 140 placed in the water tank 100.

The humidifier of the present invention may include a supply pipe 230 that supplies water held in the water tank 100 to the first humidified reservoir 300. The supply tube 230 may be placed between the water tank 100 and the first humidification reservoir 300. The supply tube 230 may temporarily hold water discharged from the water tank 100 and supplied to the first humidification reservoir 300.

A supply chamber 232 in which water is temporarily held may be formed in the supply tube 230. A sensor 234 that senses hardness of water held within the supply tube 230 may be placed on one side of the supply tube 230.

The supply pipe 230 may have a structure in which a plurality of components are coupled. The supply tube 230 may be formed by coupling a first top cover 322 and a supply tube cover 231. The supply tube cover 231 may be a structure in which the supply tube cover 231 is placed on a top side of the first top cover 322, and has the supply chamber 232 formed therein by coupling to the first top cover 322. It is also possible to form the supply tube in one structure.

The supply tube 230 may include a top supply tube 230a connected to the connector 120, a middle supply tube 230b extending downward of the top supply tube 230a, and a bottom supply tube 230c extending in a direction perpendicular to the up-down direction on a bottom of the middle supply pipe 230b.

The top supply tube 230a may be placed above the middle tray 200. The top supply tube 230a may have a structure extending upward through the first hole 204 of the middle tray 200.

The middle supply tube 230b may extend below the top supply tube 230a and may have a cylindrical shape. A tube diameter of the middle supply tube 230b may be formed to be larger than a tube diameter of the top supply tube 230a.

The bottom supply tube 230c may extend in a direction perpendicular to the vertical direction on one side of the middle supply tube 230b. The bottom supply tube 230c may form the supply chamber 232 together with the first top cover 322 placed on the top side of the first humidified reservoir 300 placed on the bottom side.

The bottom supply tube 230c may be formed between the supply tube cover 231 and the first top cover 322. The first top cover 322 may have a chamber groove 324 recessed downward in a region where the bottom supply tube 230c is formed.

A first valve 400 may be placed at an end portion of the bottom supply tube 230c. A passage formed by the supply tube cover 231 and the first top cover 322 may have a narrow passage shape, and the first valve 400 is placed at an end portion.

A passage formed at an end portion of the supply tube 230 may be opened and closed according to the placement of the first valve 400. The first valve 400 may supply or stop supplying water from the supply tube 230 to a first humidification reservoir wall 302.

The first valve 400 that supplies or stops supplying the water present in the supply tube 230 to the first humidification reservoir 300 may be placed above the first humidification reservoir 300.

A first supply hole 325 may be formed in the first top cover 322. The water present in the supply chamber 232 may move to the first humidification reservoir 300 through the first supply hole 325. The first valve 400 may open and close the first supply hole 325 to supply the water held in the supply chamber 232 to the first humidification reservoir 300.

A sensor 234 that senses a water quality of the water present within the supply chamber 232 may be placed in the supply tube 230. The sensor 234 may be placed on one side of the middle supply tube 230b. The sensor 234 may be placed above the middle supply tube 230b.

The sensor 234 may be placed above the middle supply tube 230b, and may quickly sense the water quality of the water supplied from the water tank 100. That is, when the water present in the supply chamber 232 is dropped and the water in the water tank 100 is admitted, the water quality of the water admitted from the water tank 100 may be sensed.

The first humidification reservoir 300 may have a first chamber 300a that forms a space in which water is held and water is heated.

Referring to FIGS. 12 and 13, the first humidification reservoir 300 may include a first humidification reservoir wall 302 that forms the first chamber 300a, and a heater 340 that is placed on a bottom side of the first humidification reservoir wall 302 and heats the water within the first humidification reservoir wall 302.

The first humidification reservoir wall 302 may have a cylindrical shape in which water is held. The first humidification reservoir wall 302 may be opened on the bottom side, and the heater 340 may be placed on the bottom side of the first humidification reservoir wall 302.

Referring to FIGS. 12 and 13, the first humidification reservoir wall 302 may include a bottom reservoir wall 308, a middle reservoir wall 306, and a top reservoir wall 304. The bottom reservoir wall 308, the middle reservoir wall 306, and the bottom reservoir wall 304 may be placed side by side from the bottom side to the top side.

The heater 340 may be placed on the bottom reservoir wall 308. The heater 340 may be placed to protrude inside the bottom reservoir wall 308. However, the heater 340 may be placed to be spaced radially inward from an inner peripheral surface of the bottom reservoir wall 308. The lower reservoir wall 308 may be placed to be spaced apart by a predetermined distance from the heater placed inside. A drain hole 318 may formed on one side of the bottom reservoir wall 308.

Referring to FIGS. 12 and 13, the heater 340 may include a heating element 342 that receives electricity and generates heat, and a heating plate 344 placed in contact with the heating element 342. The heating element 342 may be formed in a ring shape in which one side is opened. The heating element 342 may be placed inside the bottom reservoir wall 308.

The heating plate 344 may be placed above the heating element 342 and may transmit heat generated by the heating element 342 to the water inside the first humidification reservoir 300. The heating plate 344 may be placed to cover the heating element 342. A partial configuration may be placed to protrude inside the bottom reservoir wall 308 so as to cover the heating element 342. The heating plate 344 inside the bottom reservoir wall 308 may be placed to be spaced apart from the inner peripheral surface of the bottom reservoir wall 308.

Referring to FIG. 12, the middle reservoir wall 306 may be placed above the bottom reservoir wall 308. The middle reservoir wall 306 may be formed to have a small diameter to form a space in which a configuration of the second valve 402 is placed within the humidification module housing 410. The middle reservoir wall 306 may be formed to have a small diameter to form a space in which the first connection pipe 390 is placed within the humidification module housing 410.

Referring to FIGS. 10 to 12, the first temperature sensor 336 may be placed on one side of the middle reservoir wall 306. A recovery inflow hole 314 through which water is admitted from the second connection pipe 392 may be formed on one side of the middle reservoir wall 306. An outflow hole 316 through which water moves out to the first connection pipe 390 may be formed on one side of the middle reservoir wall 300.

The outflow hole 316 may be placed at a position higher than the recovery inflow hole 314. The first temperature sensor 336 may be placed at a position higher than the recovery inflow hole 314. The outflow hole 316 may be placed at a position higher than or equal to the first temperature sensor 336.

Thus, the first temperature sensor 336 may sense a temperature that is changed as the water supplied to the first humidification reservoir 300 is heated. In addition, the first temperature sensor 336 may sense a temperature of water held in the first chamber 300a of the first humidification reservoir 300 after some of the water held within the first humidification reservoir 300 is supplied to the second humidification reservoir 350.

The first temperature sensor 336 may sense a temperature change within the first humidification reservoir 300. The first temperature sensor 336 may sense an abnormal operation of the heater 340 within the first humidification reservoir 300.

The top reservoir wall 304 may extend above the middle reservoir wall 306. A length at which the top reservoir wall 304 extends in the up-down direction may be longer than a length at which the middle reservoir wall 306 extends in the up-down direction. A size of an internal space formed by the top reservoir wall 304 may be larger than a size of an internal space formed by the middle reservoir wall 306 and the bottom reservoir wall 308.

Referring to FIGS. 10 to 12, the first water level sensor 338 may be placed on one side of the top reservoir wall 304. The first water level sensor 338 may be placed above the first temperature sensor 336. The first water level sensor 338 may sense a water level of water within the first humidification reservoir 300. The first water level sensor 338 may sense a normal water level of the first humidification reservoir 300. Here, the normal water level of the first humidification reservoir 300 may mean a region between an upper end at which water supplied to the first humidification reservoir 300 does not exceed a set water level and a lower end which is lowest water level at which water should be present to operate the heater 340.

The first humidification reservoir 300 may include top covers 322 and 324 placed on the top side of the first humidification reservoir 300. The top covers 322 and 324 may be placed on the top side of the top reservoir wall 304.

The top covers 322 and 324 may be placed to cover at least a part of the top side of the first humidification reservoir wall 302. The top covers 322 and 324 may include a first top cover 322, and a second top cover 326 placed below the first top cover 322.

Referring to FIGS. 12 and 13, the first top cover 322 may be placed above the second top cover 326. The first top cover 322 may partition the first humidification reservoir wall 302 and the supply tube 230. The first top cover 322 may form one side of the supply chamber 232 that forms the inside of the supply tube 230.

Referring to FIGS. 12 and 13, the first top cover 322 may be formed with a first supply hole 325 through which water held in the supply chamber 232 moves. The first valve 400 may be placed above the first supply hole 325 to open and close the first supply hole 225. The first top cover 322 may be placed to be spaced upward from an upper end of the top reservoir wall 304.

The first top cover 322 may have a structure connected to the exhaust pipe 382 to be described below. The first top cover 322 may be integrally formed with the exhaust pipe 382. The first top cover 322 may have a smaller area than the second top cover 326.

The first top cover 322 may be placed to be spaced apart from the second top cover 326. That is, the first top cover 322 may be placed to be spaced apart upward from the second top cover 326.

A part of the first top cover 322 may constitute a part of the supply tube 230. The first top cover 322 may have a chamber groove 324 recessed downward in a region where the supply tube 230 is formed.

Referring to FIGS. 12 and 13, the second top cover 326 may be placed to cover the first supply hole 325 formed in the first top cover 322. A second supply hole 330 that sends the water moving through the first supply hole 325 to the first humidification reservoir 300 may be formed in the second top cover 326. The second supply hole 330 may be placed to be spaced apart from the first supply hole 325 in the direction perpendicular to the up-down direction.

Referring to FIG. 11, a supply passage 328 through which the water moving to the first supply hole 325 moves to the second supply hole 330 may be formed between the second top cover 326 and the first top cover 322. In the second top cover 326, a supply passage rib 331 that protrudes upward to form a supply passage 328 may be placed in the second top cover 326. The supply passage rib 331 may protrude above the second top cover 326 and may be placed in contact with a bottom surface of the first top cover 322.

The supply passage 328 may have a structure in which a direction in which the supply passage 328 extends is different from a direction in which the chamber groove 324 extends. As one embodiment, the direction in which the supply passage 328 extends may be formed to be perpendicular to the direction in which the chamber groove 324 extends.

Referring to FIGS. 12 and 13, a first communication hole 311a may be formed in the second top cover 326. The first communication hole 311a may connect the first humidification reservoir 300 and the second humidification reservoir 350. That is, the humidified air generated by the first humidification reservoir 300 may move to the second humidification reservoir 350 through the communication hole 311a.

The first communication hole 311a may have a structure opened in the up-down direction. Thus, the humidified air generated by the heater 340 inside the first humidification reservoir 300 may move upward through the first communication hole 311a.

The second top cover 326 may include an upper rib 333 extending upward on a periphery of the first communication hole 311a. The upper rib 333 may have a structure that protrudes upward on a periphery of the second top cover 326 in which the first communication hole 311a is formed.

An upper cover 312 may be placed above the second top cover 326. The upper cover 312 may have a structure connected to the upper rib 333. The upper cover 312 may form a communication passage 311 therein together with the upper rib 333.

The communication passage 311 may connect the first communication hole 311a formed in the first humidification reservoir 300 and a second communication hole 311b formed in the second humidification reservoir 350. The communication passage 311 may be placed above the first humidification reservoir 300. Thus, humidified air generated and rising by the first humidification reservoir 300 may move to the communication passage 311 through the first communication hole 311a.

The communication passage 311 may be formed by the upper rib 333 and the upper cover 312 of the second top cover 326. The communication passage 311 may extend in a horizontal direction to the second communication hole 311b.

A second chamber 350a that may hold water and atomize water is formed within the second humidification reservoir 350.

Referring to FIGS. 12 and 13, the second humidification reservoir 350 may include a second humidification reservoir wall 352 that forms the second chamber 350a, and a vibrator 370 that is placed below the second humidification reservoir wall 352 and vibrates to atomize water within the second humidification reservoir wall 352.

The second humidification reservoir wall 352 may have a columnar shape that forms a space therein. The vibrator 370 may be placed below the second humidification reservoir wall 352. The second humidification reservoir wall 352 may have shape in which a cross-sectional area formed in the horizontal direction increases upward.

An upper end of the second humidification reservoir wall 352 may be formed higher than an upper end of the first humidification reservoir wall 302.

The vibrator 370 may be placed on the bottom surface of the second humidification reservoir wall 352. Referring to FIG. 12, an inflow hole 358 through which the water in the first humidification reservoir wall 302 is supplied may be formed on one side of the second humidification reservoir wall 352. A recovery discharge hole 360 through which the water held within the second humidification reservoir 350 moves to the first humidification reservoir 300 may be formed on one side of the second humidification reservoir wall 350.

The inflow hole 358 may be formed at a position lower than the outflow hole 316 formed in the first humidification reservoir wall 302. The recovery discharge hole 360 may be formed at a position lower than the inflow hole 358.

Referring to FIG. 12, the vibrator 370 may be placed on the bottom surface of the second humidification reservoir 350. A recovery groove 361 forming a space in a peripheral direction in a region in which the recovery discharge hole 360 is formed may be formed on one side of the second humidification reservoir wall 352. The recovery groove 361 may be formed on one side of the vibrator 370. That is, the recovery groove 361 is placed on one side of the vibrator 370 placed on the bottom surface of the second humidification reservoir 350. That is, the recovery groove 361 may form a space at a lower end portion inside the second humidification reservoir wall 352.

The recovery discharge hole 360 may be formed at a position lower than the recovery inflow hole 314 formed in the first humidification reservoir wall 302.

Referring to FIG. 13, the second temperature sensor 364 may be placed on one side of the peripheral surface of the second humidification reservoir 350. The second temperature sensor 364 may be placed at a position higher than the recovery discharge hole 360. The second temperature sensor 364 may be placed at a position lower than or equal to the inflow hole 358.

The second temperature sensor 364 may sense a temperature of water supplied to the inside of the second humidification reservoir 350. Thus, the second temperature sensor 364 may sense whether water at a set temperature or higher is supplied to the inside of the second humidification reservoir 350.

Referring to FIGS. 9 and 13, the second water level sensor 366 may be placed on one side of the peripheral surface of the second humidification reservoir 350. The second water level sensor 366 may sense a water level of water present within the second humidification reservoir 350. The second water level sensor 366 may sense a lowest water level within the second humidification reservoir 350 at which the vibrator 350 may operate. Further, the second water level sensor 366 may sense an amount of the water held within the second humidification reservoir 350. The second water level sensor 366 may sense an optimal water level for operating the vibrator 370.

Referring to FIG. 13, a water level sensor cover 368 may be placed within the second humidification reservoir 350. The water level sensor cover 368 may be placed to be spaced apart from the second water level sensor 366 by a predetermined distance. The water level sensor cover 368 may be placed between the second water level sensor 366 and the vibrator 370. The water level sensor cover 368 may have a structure that protrudes upward from the bottom surface of the second humidification reservoir 350.

Even though a water level within the second humidification reservoir 350 is changed by the vibrator 370, the second water level sensor 366 may sense a more accurate wave level by the water level sensor cover 368.

The second communication hole 311b may be formed at an upper end portion of a peripheral wall of the second humidification reservoir 350. The second communication hole 311b may be formed at the upper end portion of the peripheral wall of the second humidification reservoir 350. The second communication hole 311b may be opened in a direction in which the first humidification reservoir 300 is placed.

Referring to FIGS. 12 and 13, the second communication hole 311b may be connected to a communication passage. The second communication hole 311b may be opened in the horizontal direction.

Two vibrators 370 may be placed on the bottom surface of the second humidification reservoir 350. Two vibrators 370 may be placed to be spaced apart from each other in the direction perpendicular to the up-down direction.

The vibrator 370 may include a vibration element 372 that generates vibration, and a vibration element cover 374 placed above the vibration element 372. The vibration element cover 374 may be placed to be in contact with the water held within the second humidification reservoir 350. The vibration element cover 374 may transmit the vibration generated by the vibration element 372 to the water held within the second humidification reservoir 350.

The vibrator 370 may generate humidified air using the water held in the second humidification reservoir 350.

Referring to FIGS. 12 and 13, the second humidification reservoir 350 may include a humidification reservoir cover 380 placed on the top side of the second humidification reservoir wall 352. The humidification reservoir cover 380 may supply humidified air generated by the second humidification reservoir 350 the first discharge passage 32.

The humidification reservoir cover 380 may include an exhaust pipe 382 that sends humidified air upward. The exhaust pipe 382 may have a structure extending upward from the humidification reservoir cover 380. The exhaust pipe 382 may have an approximately elliptical columnar shape.

Referring to FIGS. 12 and 13, the exhaust pipe 382 may be placed above a pair of vibrators 370. Thus, humidified air generated by the vibrator 370 may quickly move to the exhaust pipe 382. Further, when the water held in the second chamber 350a of the second humidification reservoir 350 is splashed upward by the vibrator 370, the water may move to the inside of the exhaust pipe 382.

An upper end of the exhaust pipe 382 may be placed to be in contact with the middle tray 200 (see FIG. 8). Here, the upper end of the exhaust pipe 382 may be placed to be in direct contact with the middle tray 200 or to be in indirect contact with the middle tray through a separate sealing member 388.

An exhaust pipe inlet 382a (or an exhaust hole) may be formed at a lower end of the exhaust pipe 382. An exhaust pipe outlet 382b may be formed at the upper end of the exhaust pipe 382. The exhaust pipe 382 may form an exhaust pipe passage 383 between the exhaust pipe inlet 382a and the exhaust pipe outlet 382b.

An air supply hole 387 may be formed on one side of the humidification reservoir cover 380. External air may be admitted into the second humidification reservoir 350 through the air supply hole 387. The air supply hole 387 may have a shape that opened upward.

The humidification reservoir cover 380 may include an air guide cover 386 that supplies air moving through the blower channel 70 to the second humidification reservoir 350. The air guide cover 386 may cause some of the air moving to the outside of the humidification module housing 410 to move into the second humidification reservoir 350 through the air supply hole 387.

The air guide cover 386 may guide the air moving to the outside of the second humidification reservoir 350 to move into the second humidification reservoir 350.

The air guide cover 386 may be placed to be spaced upward from the air supply hole 387 and have a structure extending in the horizontal direction from the exhaust pipe 382. The air guide cover 386 may extend from a peripheral wall of the exhaust pipe 382, and may be placed at a position spaced apart upward from the upper end of the second humidification reservoir wall 352.

Referring to FIGS. 12 and 13, the air guide cover 386 may include a horizontal wall 386a extending in the horizontal direction from the exhaust pipe 382 and a vertical wall 386b extending downward from an end portion of the horizontal wall 386a. The horizontal wall 386a may be placed to be spaced apart upward from the air supply hole 387 and the upper ends of the second humidification reservoir wall 352.

The vertical wall 386b may be placed to be spaced apart outward from the peripheral wall of the second humidification reservoir 350. The vertical wall 386b may be placed to be spaced apart outward from a peripheral wall of the humidification module housing 410. A lower end of the vertical wall 386b may be located to be lower than the upper end of the second humidification reservoir wall 352.

Some of the air moving through the blower channel 70 may move upward to a space between the humidification module housing 410 and the vertical wall 386b, move to the top side of the second humidification reservoir 350 along the horizontal wall 386a, and then move to a bottom of the second chamber 350a of the second humidification reservoir 350 along the air supply hole 387.

An air inflow passage 387 through which air moves may be formed below the air guide cover 386. The air inflow passage 387 may be placed above the communication passage 311. The air supply hole 387 may be placed above the communication passage 311.

The humidification reservoir cover 380 may include a bottom extension wall 384 that protrudes toward the inside of the second humidification reservoir 350 from the humidification reservoir cover 380. The bottom extension wall 384 may extend downward from the humidification reservoir cover 380. The bottom external wall 384 may have a structure that protrudes downward in a region where the exhaust pipe 382 is formed.

Referring to FIGS. 12 and 13, the bottom extension wall 384 may be placed between the exhaust pipe inlet 382a and the air supply hole 387. The bottom extension wall 384 may have a shape bent toward the exhaust pipe inlet 382a when viewed from the top side. Thus, air admitted into the second humidification reservoir 350 through the air supply hole 387 may move along the bottom of the second chamber 350a and an inner circumferential surface of the second humidification reservoir wall 352.

The bottom extension wall 384 may extend downward between the exhaust pipe inlet 382a and the air supply hole 387. The bottom extension wall 384 may allow the air admitted into the second humidification reservoir 350 through the air supply hole 387 to move to the bottom of the second chamber 350a.

The bottom extensional wall 384 may not be placed in a direction in which the second communication hole 311b is formed. That is, the bottom extensional wall 384 may not be placed in a direction in which the second communication hole 311b is opened. Thus, humidified air admitted into the second humidification reservoir 350 through the second communication hole 311b may move to the exhaust pipe inlet 382a of the exhaust pipe 382.

The bottom extensional wall 384 may include a first bottom extensional wall 384a that extends downward from the exhaust pipe 382 at a portion where the air supply hole 387 is formed, a second bottom extensional wall 384b that is placed on one side of the first bottom extensional wall 384a, and a third bottom extensional wall 384c that is placed on the other side of the first bottom extension wall 384a.

The second bottom extensional wall 384b and the third bottom extensional wall 384c may be placed in opposite directions to each other. The second bottom extensional wall 384b and the third bottom extensional wall 384c may be placed to face each other.

A lower end of the first bottom extensional wall 384a may be formed lower than an upper end of the water level sensor cover 368. A lower end of each of the second bottom extensional wall 384b and the third bottom extensional wall 384c may be formed higher than the upper end of the water level sensor cover 368. The water level sensor cover 368 may be placed below the second bottom extensional wall 384b or the third bottom extensional wall 384c.

The lower end of the first bottom extensional wall 384a may be located lower than the lower end of the second bottom extensional wall 384b or the lower end of the third bottom extensional wall 384c. A hole through which the humidified air moving in the second communication hole 311b moves into the exhaust pipe 382 may be formed in a region facing the second communication hole 311b.

The humidification reservoir cover 380 may be integrally formed with the first top cover 322.

The first connection pipe 390 may supply water from the first humidification reservoir 300 to the second humidification reservoir 350. One side of the first connection pipe 390 may be connected to the first humidification reservoir 300, and the other side may be connected to the second humidification reservoir 350. The first connection pipe 390 may connect the outflow hole 316 of the first humidification reservoir 300 and the inflow hole 358 of the second humidification reservoir 350. The first connection pipe 390 may be placed so as to be lowered downward toward the inflow hole 358 from the outflow hole 316.

The first connection pipe 390 may extend along a circumferential periphery of the first humidification reservoir wall 302. The inflow hole 358 and the outflow hole 316 may be placed in different directions. Thus, the first connection pipe 390 may have a structure that extends along the periphery of the first humidification reservoir wall 302.

The first connection pipe 390 may be formed to be longer than the second connection pipe 392. The second valve 402 may be placed in the first connection pipe 390. The second valve 402 may be placed closer to the first humidification reservoir 300 than the second humidification reservoir 350. The second valve 402 may be placed closer to the outflow hole 316 than the inflow hole 358.

The second connection pipe 392 may supply water in the second humidification reservoir 350 to the first humidification reservoir 300. A pump 404 may be placed in the second connection pipe 392. The second connection pipe 392 may connect the recovery discharge hole 360 of the second humidification reservoir wall 352 and the recovery inflow hole 314 of the first humidification reservoir wall 302.

The recovery discharge hole 360 may be located lower than the recovery inflow hole 314. The pump 404 may be placed above the recovery discharge hole 360 and the recovery inflow hole 314.

The second connection pipe 392 may include a pump inflow pipe 392a connecting the second humidification reservoir 350 and the pump 404, and a pump outflow pipe 392b connecting the pump 404 and the first humidification reservoir 300. The pump 404 may be placed on one side of the second humidification reservoir 350.

The humidifier may include a drain pipe 394 that drains the water present in the first humidification reservoir 300, and a drain pipe valve 396 that is placed at an end portion of the drain pipe 394, and opens and closes the drain pipe 394. Referring to FIG. 11, the drain pipe 394 may be connected to one side of the first humidification reservoir 300. The drain pipe valve 396 may be placed at the end portion of the drain pipe 394.

The drain pipe 394 may discharge the water held in the first humidification reservoir 300 to the outside. Referring to FIG. 10, the drain pipe 394 may extend to the outside of the humidification module housing 410.

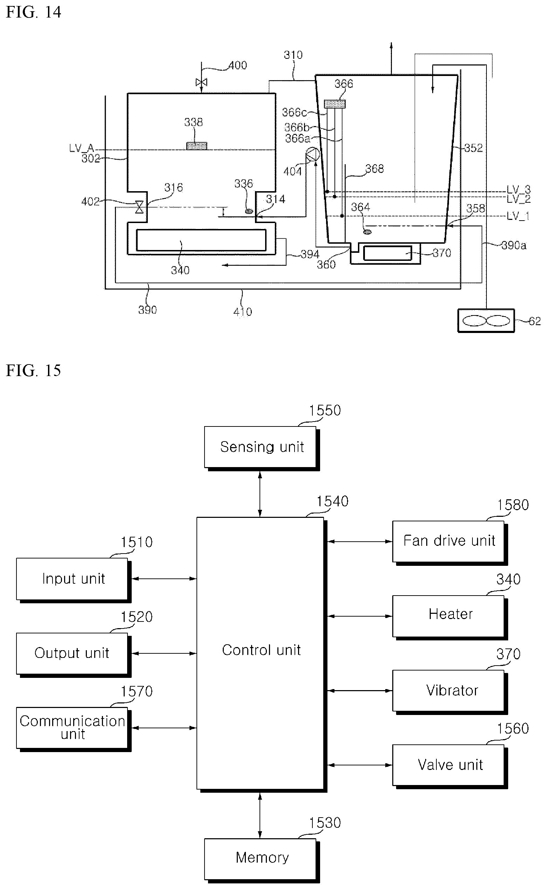

FIG. 14 is a schematic diagram for describing a specific connection relationship between the first humidification reservoir and the second humidification reservoir, and a placement relationship of internal components according to an embodiment of the present invention.

Referring to FIG. 14, various sensors and valves placed in each of the first humidification reservoir 300 and the second humidification reservoir 350, and operations of the first humidification reservoir 300 and the second humidification reservoir 350 will be described.

The first humidification reservoir 300 may heat the water supplied from the water tank 100. The water in the water tank 100 may be supplied to the top side of the first humidification reservoir 300. The first valve 400 may open the supply tube 230 to supply the water to the first humidification reservoir 300.

The first valve 400 may be operated based on the water level sensed by the first water level sensor 338. The first valve 400 may open the supply tube 230 when the water level sensed by the first water level sensor 338 is lower than a set water level. The set water level as a water level that serves as a reference for opening and closing the supply tube 230 may also be referred to as a water supply level or a reference water level LV-A.

The first valve 400 may close the supply tube 230 when the water level sensed by the first water level sensor 338 is equal to or higher than the set water level. When the second valve 402 opens the first connection pipe 390, the first valve 400 may close the supply tube 230. Thus, as the water that moves through the first connection pipe 390, water of which heating is completed in the first humidification reservoir 300 may move.

The first valve 400 may open the supply tube 230 when a water level sensed by the sensor 234 is equal to or higher than a set water level. The sensor 234 may measure the water quality by measuring electrical conductivity of water. That is, the sensor 234 may sense a concentration of ions by measuring the electrical conductivity of the water. Thus, water having an improved water quality by passing through the water softener 140 may be supplied to the first humidification reservoir 300.

When water is supplied to the first humidification reservoir 300 at a predetermined water level and the first valve 400 closes the supply tube 230, the heater 340 may operate. The heater 340 may heat the water held in the first humidification reservoir 300. Humidified air generated by operating the heater 340 may move to the second humidification reservoir 350 through the communication pipe 310.

When the heater 340 operates, the blower fan 62 may operate. That is, when the heater 340 heats the water held in the first humidification reservoir 300, the blower fan 62 may operate to supply air to the second humidification reservoir 350. The blower fan 62 may supply air to the second humidification reservoir 350 to cool the inside of the second humidification reservoir 350.

The heater 340 may operate until the temperature of the water sensed by the first temperature sensor 336 reaches 100 degrees. The water held in the first humidification reservoir 300 may be heated to a boiling point to be sterilized. The second valve 402 may open the first connection pipe 390 when the temperature of the water sensed by the first temperature sensor 336 reaches 100 degrees. The second valve 402 may open the first connection pipe 390 after the temperature of the water sensed by the first temperature sensor 336 reaches 100 degrees.

The first temperature sensor 336 may be placed at a position equal to or lower than the outflow hole 316. Thus, after water in the second humidification reservoir 350 is discharged to the first connection pipe 390, a temperature of remaining water may be sensed.

When the second valve 402 is opened, the water held in the first humidification reservoir 300 moves to the second humidification reservoir 350 by gravity. When the second valve 402 is opened, water may move until the water levels of the first humidification reservoir 300 and the second humidification reservoir 350 are equal to each other. When the second valve 402 is opened, the water level of the first humidification reservoir 300 may be lowered up to a height of the outflow hole 316 formed in the first humidification reservoir 300.

The second valve 402 may open the first connection pipe 390 when the second water level sensor 366 is lower than an optimal height at which the vibrator 370 of the second humidification reservoir 350 operates. The second valve 402 may open the first connection pipe 390 when there is a difference between the water levels of the first humidification reservoir 300 and the second humidification reservoir 350 sensed by the first water level sensor 338 and the second water level sensor 366.

The second valve 402 may be opened for a set time. That is, by considering a time when the water levels of the first humidification reservoir 300 and the second humidification reservoir 350 are equal to each other, the second valve 402 may be opened for the set time, and when the set time elapses, the first connection pipe 390 may be closed. By considering a time for which the water in the first humidification reservoir 300 moves to the second humidification reservoir 350, the second valve 402 may be opened for the set time.

While the second valve 402 is opened, the blower fan 62 may operate. The air admitted to the second humidification reservoir 350 by operating the blower fan 62 may cool the water admitted to the second humidification reservoir 350 through the first connection pipe 390.

The second temperature sensor 364 may sense the temperature of the water held in the second humidification reservoir 350. The second temperature sensor 364 may sense the temperature of the water admitted to the second humidification reservoir 350 through the inflow hole 358. The vibrator 370 may operate when the temperature of the water sensed by the second temperature sensor 364 is equal to or lower than a set temperature. The set temperature for operating the vibrator 370 may be set by considering prevention of damage to the vibrator 370.

The second temperature sensor 364 may be placed at the same height as the inflow hole 358 or at a lower height than the inflow hole 358. Thus, a temperature of the water admitted through the inflow hole 358 may be sensed.

The second water level sensor 366 may sense the water level of the second humidification reservoir 350. The second water level sensor 366 may sense a lowest water level of the second humidification reservoir 350 at which the vibrator 350 does not operate. When the water level of the second humidification reservoir 350 sensed by the second water level sensor 366 is formed to be lower than the set water level, the operation of the vibrator 370 may be stopped. The lowest water level at which the operation of the vibrator 370 is stopped may be determined based on the water level of the water held in the second humidification reservoir 350 relative to a vibration level of the vibrator 370.

The second water level sensor 366 may sense a water level at which the vibrator 370 operates optimally. The second water level sensor 366 may sense an upper limit water level and a lower limit water level at which the operation of the vibrator 370 may be optimal at a position at which the water in the second humidification reservoir 350 is higher than the lowest water level at which the operation of the vibrator 370 is stopped.

The second water level sensor 366 may be a sensor that may sense a plurality of levels. For example, the second water level sensor 366 may be a three-channel (366a, 366b, and 366c) sensor that may sense water levels of three levels LV_1, LV_2, and LV_3. A first channel 366a may sense a first water level LV_1. The first water level LV_1 may be a lowest water level that may be sensed by the second water level sensor 366.

A second channel 366b may sense a second level LV_2, and a third channel 366c may sense a third level LV_3. The second level LV_2 may be a water level higher than the first level LV_1, and the third level LV_3 may be a water level higher than the second level LV_2.

The first water level LV_1 may be the lowest water level. The second water level LV_2 and the third water level LV_3 may be a management water level range corresponding to the vibrator 370. The second water level LV_2 may be a lower limit water level of the management water level range, and the third water level LV_3 may be an upper limit water level of the management water level range.

In the second humidification reservoir 350, the water level sensor cover 368 is placed between the second water level sensor 366 and the vibrator 370, and stable water level sensing is thus possible even during the operation of the vibrator 370.

The vibrator 370 may operate when the water level sensed by the second water level sensor 366 is equal to higher than the set water level. The vibrator 370 may include two vibration elements 372. Thus, two vibration elements 372 may operate individually. Two vibration elements 372 may operate selectively.

When the vibrator 370 operates, the blower fan 62 may operate. Thus, humidified air generated by the vibrator 370 may be discharged to the outside of the humidifier together with the air moving from the second humidified water tank 350 to the first discharge passage 32 by the blower fan 62. The blower fan 62 may activate the movement of the humidified air generated by the vibrator 370.

The vibrator 370 and the heater 340 may operate simultaneously. In this case, the humidified air generated by the first humidification reservoir 300 move to the first discharge passage 32 through the second humidification reservoir 350, and the humidified air generated by the second humidification reservoir 350 may also move to the first discharge passage 32.

The pump 404 may allow the water in the second humidification reservoir 350 to move to the first humidification reservoir 300. The pump 404 may operate after the operation of the vibrator 370 is stopped. The pump 404 may operate after the second valve 402 closes the first connection pipe 390. When the pump 404 operates, the first valve 400 may close the supply tube 230. When the pump 404 operates, the operation of the blower fan 62 may be stopped.

The recovery discharge hole 360 may be formed on one side of a height at which the vibrator 370 is located. The recovery discharge hole 360 may be placed at a lower position than the inflow hole 358 formed in the second humidification reservoir 350. The pump 404 may be placed at a position higher than positions where the recovery discharge hole 360 and the recovery inflow hole 314 are formed. Thus, the water in the second humidification reservoir wall 352 may be sucked and the water may be supplied to the first humidification reservoir 300.

The pump 404 may operate in consideration of the water level of the first humidification reservoir 300 sensed by the first water level sensor 338 and the water level of the second humidification reservoir 350 sensed by the second water level sensor 366. In a state where the water level of the first humidification reservoir 300 is equal to or higher than a proper water level, the operation of the pump 404 may be prevented.

The water held in the first humidification reservoir 300 may be discharged to the outside through the drain pipe 394. When the drain pipe valve 396 opens the drain pipe 394, the water held in the first humidification reservoir 300 may be discharged to the outside. The drain hole 317 formed in the first humidification reservoir 300 may be placed at a position where the heater 340 is placed. Water stored between the heater 340 and the first humidification reservoir 300 may move to the drain pipe 394 through the drain hole 318.

FIG. 15 is a block diagram of a humidifier according to an embodiment of the present invention.

Referring to FIG. 15, a humidifier according to an embodiment of the present invention may include a control unit 1540 that controls an overall operation of the humidifier to provide a humidification and/or air purification function.

At least a part of an internal block of the humidifier illustrated in FIG. 15, such as a control unit 1540, may be placed within a casing 10. For example, the control unit 1540 may be mounted on a circuit board 76 placed in a control box 74.

A blower fan 62 for controlling a flow in which sucked air is purified through a filter 50 and then discharged may be installed within the casing 10.

The control unit 1540 may control a revolution per minute (RPM) of the blower fan 62 in response to an operation mode. A fan drive unit 1580 may drive the blower fan 62 according to the control of the control unit 1540.

A fan motor 64 may rotate the blower fan 62 according to a control signal received by the fan drive unit 1580. The fan drive unit 1580 may control the rotation of the blower fan 62, and the operation of the fan drive unit 1580 is controlled by the control unit 1540. The fan drive unit 1580 may include a power conversion device such as an inverter to operate the fan motor 64.

The humidifier may include a heater 340, a vibrator 370, and a valve unit 1560. The control unit 1540 may control the heater 340, the vibrator 370, and the valve unit 1560.

For example, the valve unit 1560 may include a first valve 400 placed in a supply tube 230 that connects a water tank 100 and a first humidification reservoir 300, and a second valve 402 that connects the first humidification reservoir 300 and a second humidification reservoir 350, and opens and closes a first connection pipe 390.

The control unit 1540 may open the supply tube 230 when a water level sensed by a first water level sensor 338 is lower than a reference water level LV-A. The control unit 1540 may control the first valve 400 in an open state to open the supply tube 230.

When the supply tube 230 is opened, water may be supplied from the water tank 100 to the first humidification reservoir 300. The control unit 1540 may operate the heater 340 to heat water in a first chamber 300a. The heater 340 may heat the water in the first chamber 300a formed in the first humidification reservoir 300.

The control unit 1540 may control the second valve 402 in response to a water level sensed by a second water level sensor 366.

The second water level LV_2 may be a low water level at which water supplying is started, and the third water level LV_3 may be a full water level at which water supplying is finished. The first water level LV_1 may be a poor water level at which the operation is stopped.

The control unit 1540 may control the second valve 402 in the open state to open the first connection pipe 390. When the first connection pipe 390 is opened, water heated by operating the heater 340 in the first humidification reservoir 300 moves to the second humidification reservoir 350.

Meanwhile, the first humidification reservoir 300 and the second humidification reservoir 350 may be connected through a communication pipe 310. Vapor generated by operating the heater 340 in the first humidification water reservoir 300 moves to the second humidification reservoir 350 through the communication pipe 310.

The control unit 1540 may operate the vibrator 370 in response to the operation mode including the humidification function. The control unit 1540 may control a duty of the vibrator 370 in response to the operation mode. The control unit 1540 may generate humidified air by operating the vibrator 370.

In the second humidification reservoir 350, an air supply hole 387 through which the air moving by the blower fan 62 is supplied to the second chamber 350a may be formed. In the second humidification reservoir 350, an exhaust hole 382a that discharges humidified air generated by the second chamber 350a to the outside may be formed. An exhaust pipe 382 may send the humidified air generated by the second humidification reservoir 350 to a first discharge passage 32. The exhaust hole 382a may be formed at a lower end of the exhaust pipe 382.

The humidified air discharged from the second humidification reservoir 350 may move to the first discharge passage 32. Meanwhile, air filtered by the filter 50 may move to a second discharge passage 34. In the mixture passage 13, the air moving through the first discharge passage 32 and the air moving through the second discharge passage 34 may be mixed together.

The control unit 1540 may operate the heater 340 in response to the operation mode. The operation mode including the humidification function may include a mode in which the heater 340 is driven according to a condition and a mode in which the heater 340 is always driven.

The control unit 1540 may control a duty of the heater 340 in response to the operation mode. Further, the control unit 1540 may control an output of the heater 340 in response to the operation mode.

The control unit 1540 may control the humidifier according to a humidification amount set for each operation mode. The control unit 1540 may control the blower fan 62, the heater 340, and the vibrator 370 based on the humidification amount.

The control unit 1540 may recover residual water by operating the pump 404 when a humidification operation ends.

Meanwhile, the humidifier may include a memory 1530, a sensing unit 1550, and a communication unit 1570.

The memory 1530 may store data for operation control of the humidifier, data sensed or measured through the sensing unit 1550 during the operation, and data received through the communication unit 1570. Meanwhile, the humidifier may include a buffer for temporarily storing data, and the buffer may be included in the control unit 1540 or the memory 1530.

The control unit 1540 may process various types of data received through the communication unit 1570 to update the memory 1530. For example, when the data input through the communication unit 1570 is updated data for an operation program pre-stored in the memory 1530, the data may be used to update the memory 1530 and when the input data is a new operation program, the data may further be stored in the memory 1530.

The sensing unit 1550 may include one or more sensors to measure a water temperature, a water level, a humidity, or the like, or to sense an operating state of the humidifier.

For example, the sensing unit 1550 may include a first water level sensor 338 that senses the water level of the water held within the first humidification reservoir 300, and a first temperature sensor 336 that senses the temperature of the water held within the first humidification reservoir 300, a second water level sensor 366 that senses the water level of the water held within the second humidification reservoir 350, and a second temperature sensor 364 that senses the temperature of the water held within the second humidification reservoir 350.

For example, the sensing unit 1550 may include a humidity sensor (not illustrated).

The control unit 1540 may drive the vibrator 370 according to the water levels sensed by the water level sensors 338 and 366, a humidity of the indoor space sensed by the humidity sensor, a desired humidity set by the user, an amount of fumes, the operation mode, and the like.

The control unit 1540 may control a flow of data input or output to the humidifier, and generate and apply a control command based on the data input from the sensing unit 1550.

Meanwhile, the humidifier may be communicatively connected to other devices such as a server, a home appliance, a terminal, and the like through the communication unit 1570 to transmit and receive data.

The input unit 1510 may receive various user commands related to the operation of the humidifier, and may transfer a control signal corresponding to the input command to the control unit 1540. The input unit 1510 may include a touch pad, a physical button, and the like.

The output unit 1520 may include a display device such as the display 30 a light emitting diode (LED) (not illustrated), or the like. For example, the output unit 1520 may display information such as an operation state of the humidifier, an operating state related to occurrence of an error, and the like, an indoor temperature, a target temperature, or the like.

The output unit 1520 may include an audio device such as a speaker (not illustrated), a buzzer (not illustrated), or the like. For example, the output unit 1520 may output an effect sound for the operation state of the humidifier, and output a predetermined warning sound when an error occurs.

The control unit 1540 may be connected to each component provided in the humidifier. For example, the control unit 1540 may mutually transmit and/or receive signals to and/or from each component provided in the humidifier, and control an overall operation of each component.

The control unit 1540 may include at least one processor, and control an overall operation of the humidifier by using the processor included therein. Here, the processor may be a general processor such as a central processing unit (CPU). The processor may be a dedicated device such as ASIC or another hardware based processor.

Meanwhile, the control unit 1540 may control the overall operation of the humidifier, and control a humidification function, an air purification function, and various sterilization functions.

The humidifier may provide a plurality of operation modes. For example, it is possible to provide a humidification operation mode that provides the humidification function, an air purification mode that provides the air purification function, and a humidification and purification mode that provides both the humidification function and the air purification function. Further, the humidification operation mode may include a cozy humidification mode in which the heater 340 is always driven and a pure humidification mode in which the heater 340 is driven according to conditions.

In the present invention, the humidification operation which provides a humidification function may be the humidification operation mode, the humidification purification mode, the cozy humidification mode, or the pure humidification mode.

Meanwhile, each operation mode may include a plurality of operation steps in which control options of the blower fan 62, the heater 340, and the vibrator 370 are set differently. For example, the plurality of operation steps may include a bedtime operation step, a weak operation step, a medium operation step, a strong operation step, and a turbo operation step in order of operation intensity.

Further, the humidifier may provide one or more sterilization modes.

In the flow path sterilization mode, the control unit 1540 may supply water up to the reference water level LV_A again to the first chamber 300a, and operate the heater 340 to boil the water within the first chamber 300a.

Vapor generated by boiling water by the operation of the heater 340 may move to the first discharge passage 32 via the communication pipe 310 and the second humidification reservoir 350. As a result, the first discharge passage 32 through which the humidified air passes may be sterilized. The vapor generated by the boiled water has sterilization power and remove harmful bacteria and viruses from the first discharge passage 32. Sanitation and reliability may be improved by sterilizing the passage through which the humidified air passes with sterilizing vapor.

Meanwhile, the control unit 1540 may operate the heater 340 at a first output, boil the water within the first chamber 300a, and then switch the output of the heater 340 to a second output lower than the first output. In addition, the control unit 1540 may maintain a heating state in which the heater 340 is operated at the second output for a predetermined time.

In order to effectively sterilize the first discharge passage 32, it is necessary to continuously generate and send the sterilizing vapor. Even after the water is boiled, the heater 340 may be driven to continuously generate vapor.

After the water is boiled, if the output of the heater 340 is lowered and maintained for a predetermined time, a vapor generation state may also be maintained. Therefore, power consumption may be reduced by lowering the output of the heater 340, and waste of the water within the first chamber 300a boiling off too quickly may be prevented.

The sterilization operation of the passage sterilization mode may be constituted by a process of supplying water up to the reference water level LV_A to the first chamber 300a, and operating the heater 340 at the first output, boiling the water within the first chamber 300a, and then switching an output of the heater 340 to a second output, and maintaining the heating state for a predetermined time to be performed a predetermined number of times.

The control unit 1540 may control the sterilization operation to be repeatedly performed. For example, when the sterilization operation is performed twice, an area of 75 to 80% of the first discharge passage 32 may be sterilized by 99.9% or more.

Meanwhile, when the water temperature of the first humidification reservoir 300 reaches 100 degrees Celsius, the control unit 1540 may determine this state as a boiling state in which water is boiled and vapor is generated.

Even when the temperature sensed by the first temperature sensor 336 is less than 100 degrees Celsius due to a sensing error or the like of the first temperature sensor, there may be the boiling state. Therefore, when the water temperature reaches a first temperature (e.g., 97 degrees) and then is maintained for a preset reference time, the control unit 1540 may determine this state as the boiling state.

Meanwhile, when the user sets the passage sterilization mode, the passage sanitization mode may be preferentially performed before the humidification operation. By first sterilizing the passage with vapor generated by boiling water before the humidification operation, clean humidified air may be discharged.

Hereinafter, a passage sterilization mode will be described in more detail with reference to drawings.

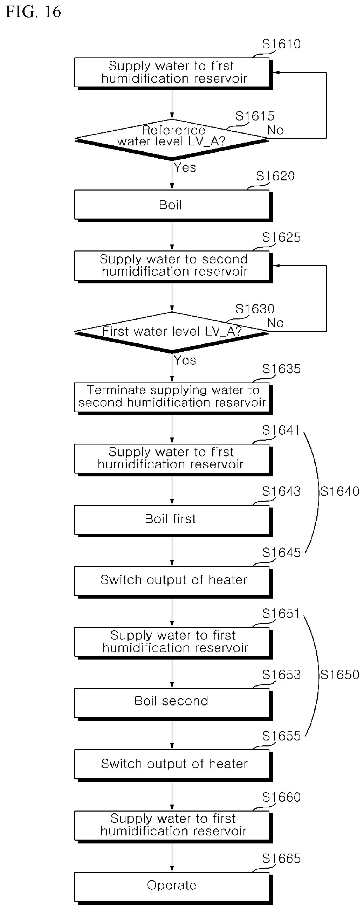

FIG. 16 is a flowchart illustrating a method for operating a humidifier according to an embodiment of the present invention.

Referring to FIG. 16, in the passage sterilization mode, the control unit 1540 may control the first valve 400 to supply water to the first chamber 300a of the first humidification reservoir 300 (S1610). The control unit 1540 may supply water to the reference water level LV_A (S1615).

During an operation of the passage sterilization mode, the display 30 may display information indicating that the passage sterilization mode is operating.

When the reference water level LV_A is sensed by the first water level sensor 338 (S1615), the control unit 1540 may control the first valve 400 to terminate water supply to the first humidification reservoir 300.

Meanwhile, when the reference water level LV_A is not sensed by the first water level sensor 338, and there is an empty water state in which the water level of the second humidification reservoir 350 is lower than the first water level LV_1, closing the first valve 400 may be delayed. When the reference water level LV_A is not sensed by the first water level sensor 338, and the water level of the second humidification reservoir 350 is lower than the first water level LV_1, there may be an initial state or a state in which water is insufficient. Thus, by delaying the closing of the first valve 400, water may be supplied a little more.

Meanwhile, the control unit 1540 may operate the heater 340 to boil water within the first chamber 300a. When boiling water, the control unit 1540 may cause the heater 340 to operate at a first output.

When the heater 340 operates, the blower fan 62 may operate. The blower fan 62 may be placed between the first humidification reservoir 300 and the filter 50. The blower fan 62 may be placed between the second humidification reservoir 350 and the filter 50.

When the heater 340 may heat the water held in the first humidification reservoir 300, the blower fan 62 may operate to supply air to the second humidification reservoir 350. Further, air movement from the second humidification reservoir 350 to the first discharge passage 32 may occur.

Meanwhile, when the blower fan 62 starts to operate, an operating state may be maintained during the operation of the passage sterilization mode. As a result, the air movement may be continuously formed during the passage sterilization mode.

When the water is determined to be in a boiling state, the control unit 1540 may control the second valve 402 to open the first connection pipe 390, thereby supplying water to the second humidification reservoir 350 up to the first water level LV_1 (S1625).

When the reference water level LV_A is sensed by the second water level sensor 366 (S1630), the control unit 1540 may control the second valve 402 to close the first connection pipe 390, thereby terminating water supply to the second humidification reservoir 350.

As a result, boiled water may remain in the second humidification reservoir 350. A state of the filter 50 may be checked based on a water temperature of the second humidification reservoir 350 sensed by the second temperature sensor 364.

The filter 50 may be placed below the second humidification reservoir 350 and the blower fan 62, and may be located upstream of the second humidification reservoir 350 upstream of air current.

When there is an abnormal situation such as the filter 50 being mounted in a state where a plastic packaging is not removed, water temperature data within the second humidification reservoir 350 shows a different trend from water temperature data in a normal situation. In addition, in the abnormal situation, a phenomenon occurs in which the water temperature data within the second humidification reservoir 350 does not fall below a constant temperature within a check time. Reflecting such a phenomenon, it is possible to set a check time for monitoring a change in water temperature and a check reference temperature that serves as a criterion for determining the abnormal situation.

After the closing of the first connection pipe 390, when a state in which the water temperature of the second humidification reservoir 350 is equal to or higher than a preset check reference temperature is maintained for the check time or longer, the control unit 1540 may determine that the filter state needs to be checked.

Further, after the closing of the first connection pipe 390, when the state in which the water temperature of the second humidification reservoir 350 is equal to or higher than the check reference temperature is maintained for the check time or longer, the control unit 1540 may control the output unit 1520 or the communication unit 1570 to output a filter state check requirement notification.

By effectively determining the state of the filter 50 in the passage sterilization mode performed before the humidification operation, both the humidification function and the air purification function with high reliability may be provided.

Meanwhile, an air volume of the air moving through the second discharge passage 34 may be larger than an air volume of the air moving through the first discharge passage 32. Only when the air volume of the second discharge passage 34 is large, the air movement may be formed smoothly.

The control unit 1540 may control a first sterilization operation to be performed (S1640).

The control unit 1540 may supply water up to the reference water level LV_A to the first chamber 300a (S1641), cause the heater 340 to operate at the first output, boils the water within the first chamber 300a (S1643), and then switch an output of the heater 340 to a second output lower than the first output to maintain a heating state for a predetermined time (S1645). Water boiling included in the first sterilization operation (S1643) may be referred to as first boiling.

The control unit 1540 may control a second sterilization operation to be performed (S1650).

The control unit 1540 may supply water up to the reference water level LV_A to the first chamber 300a (S1651), cause the heater 340 to operate at the first output, boils the water within the first chamber 300a (S1653), and then switch an output of the heater 340 to a second output lower than the first output to maintain a heating state for a predetermined time (S1655). Water boiling included in the second sterilization operation (S1653) may be referred to as second boiling.

Meanwhile, when a passage sterilization operation is completed (S1650), water is supplied until the water level of the first humidification reservoir 300 reaches the reference water level LV_A (S1660). As a result, once boiled water remains in the second humidification reservoir 350, and water is present in the first humidification reservoir 300 up to the reference water level LV_A, the humidification operation may be switched on more quickly (S1665).

Thereafter, the humidified air may be generated using the heated water, thereby providing clean and comfortable humidified air.

Further, with seamless control logic in which the passage sterilization and the humidification operation are connected, efficiency is enhanced.

Meanwhile, when the vibrator 370 operates during the operation of the passage sterilization mode, unnecessary power consumption occurs, and the temperature falls faster, which may be inefficient in boiling water to generate vapor. Thus, more preferably, the vibrator 370 may maintain an off state during the operation of the passage sterilization mode.

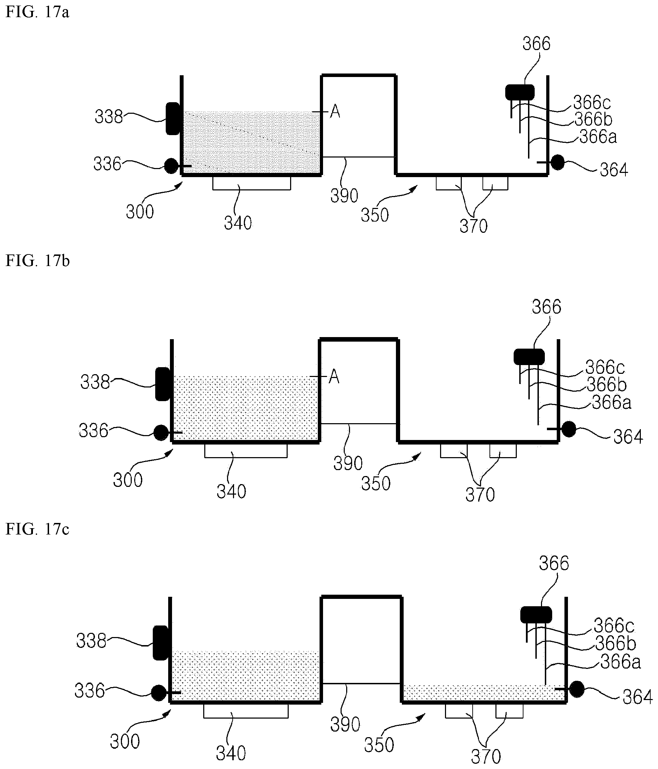

FIGS. 17A to 17I are diagrams referred to in the description of a passage sterilization mode according to an embodiment of the present invention.

Referring to FIG. 17A, water may be supplied up to the reference water level LV_A of the first humidification reservoir 300.

When the water level of the first humidification reservoir 300 reaches the reference water level LV_A, the water supply may be terminated. If the water level of the second humidification reservoir 350 is in a poor water state below the first water level LV_1, the water supply may be delayed by several seconds without immediately terminating the water supply even if the reference water level LV_A is sensed.

Referring to FIG. 17B, the heater 340 may be operated at the first output to boil the water within the first chamber 300a. For example, when the temperature data sensed by the first temperature sensor 336 reaches a preset first temperature (e.g., 97 degrees) and then held for a reference time, this state may be judged as a boiling state.

Referring to FIG. 17C, water may be supplied to the first water level LV_1 of the second humidification reservoir 350. When the first connection pipe 390 is opened, heated water is admitted into the second humidification reservoir 300, and the water level of the first humidification reservoir 300 may be lowered.

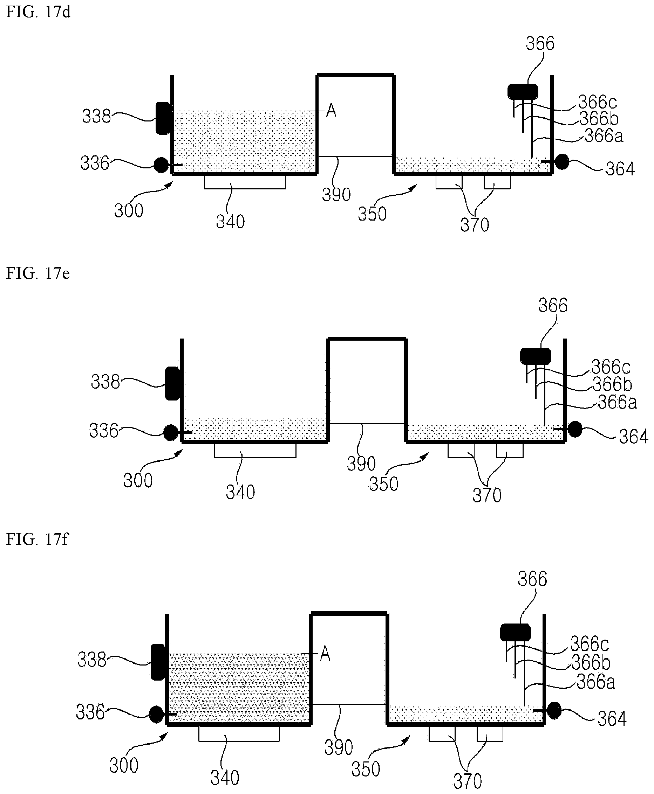

Referring to FIG. 17D, water may be supplied to the reference water level LV_A again to the first chamber 300a, and the heater 340 may be operated at the first output to boil water.

Referring to FIG. 17E, after the water is boiled, the output of the heater 340 may be switched to a second output lower than the first output to maintain the heating state for a predetermined time.

When the predetermined time elapses and the passage sterilization operation is completed once, water may be supplied until the water level of the first humidification reservoir 300 reaches the reference water level LV_A as illustrated in FIG. 17F.

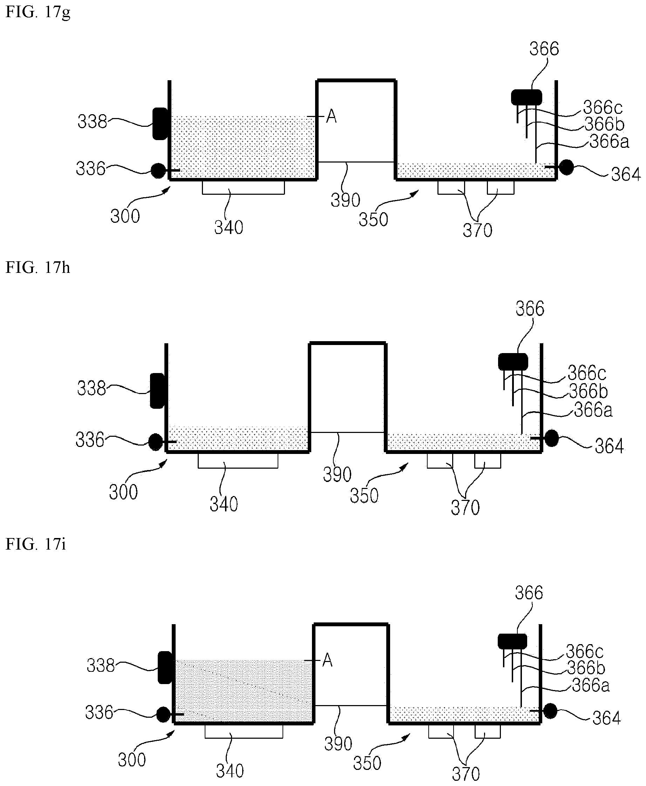

Referring to FIG. 17G, the heater 340 may be operated at the first output to boil water again.

Further, referring to FIG. 17H, after the water is boiled, the output of the heater 340 may be switched to a second output lower than the first output to maintain the heating state for a predetermined time.

When the predetermined time elapses and the passage sterilization operation is completed twice, water may be supplied until the water level of the first humidification reservoir 300 reaches the reference water level LV_A as illustrated in FIG. 17I.

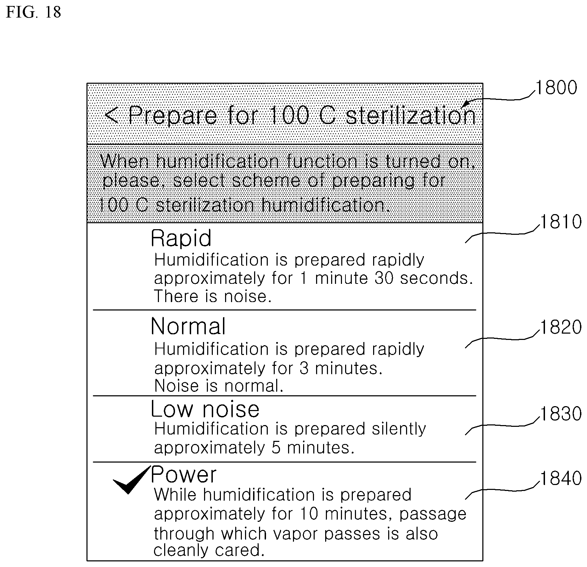

FIG. 18 is a diagram referred to in the description of passage sterilization mode setting of the humidifier according to an embodiment of the present invention.

Referring to FIG. 18, a user may execute an application with his/her mobile terminal to set a humidification function preparation time.

A humidification function setting screen 1800 may include a plurality of setting items 1810, 1820, 1830, and 1840.

The user may select any one of a "rapid" item 1810, a "normal" item 1820, and a "low noise" item 1830 to set the humidification function.

The humidification function preparation time is lengthened in the order of the "rapid" item 1810, the "normal" item 1820, and the "low noise" item 1830, and noise may be reduced in the order of "rapid" item 1810, the "normal" item 1820, and the "low noise" item. For example, a humidification operation corresponding to the "rapid" item 1810 may have a shortest preparation time and a loudest noise.

In addition, if the user selects a "power" item 1840 with a longest preparation time, the use of the passage sterilization mode may be set. When the passage sterilization mode is set, the passage sterilization mode may be first performed in a pre-operation before the humidification operation.

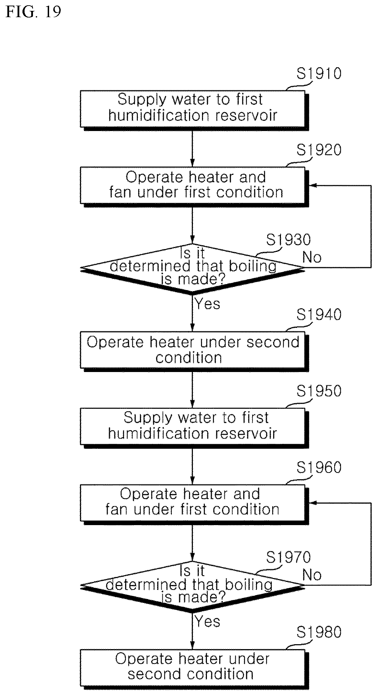

FIG. 19 is a flowchart illustrating a method for operating a humidifier according to an embodiment of the present invention.

Referring to FIG. 19, the control unit 1540 may supply water to the first humidification reservoir 300 up to the reference water level LV_A (S1910).

The control unit 1540 may operate the heater 340 and the blower fan 62 under a first condition to boil water within the first humidification reservoir 300 (S1920). A first condition of the heater 340 may be a first output, and a first condition of the blower fan 62 may be a first RPM.

When the water temperature of the first humidification reservoir 300 reaches 100 degrees Celsius or is lower than 100 degrees Celsius (e.g., 97 degrees) is maintained for a preset reference time, the control unit 1540 may determine that this state is a boiling state (S1930).

When the state is determined as the boiling state (S1930), the control unit 1540 may operate the heater 340 under a second condition (S1940). The second condition of the heater 340 may be a second output lower than the first output. The blower fan 62 may maintain the first condition.

By operating the heater 340, the water within the first humidification reservoir 300 may be boiled to generate vapor. The vapor may move to the first discharge passage 32 to sterilize the first discharge passage 32 through which humidified air passes during the humidification operation.

After the heater 340 operates under the second condition for a predetermined time (S1940), the control unit 1540 may supply water to the first humidification reservoir 300 up to the reference water level LV_A (S1950).

In addition, an operation state of the heater 340 may be switched to the first condition again to boil water (S1960). The blower fan 62 may still maintain the first condition (S1960).

When the state is determined as the boiling state (S1970), the control unit 1540 may operate the heater 340 under the second condition and maintain the heater 340 in the heating state for a predetermined time (S1980).

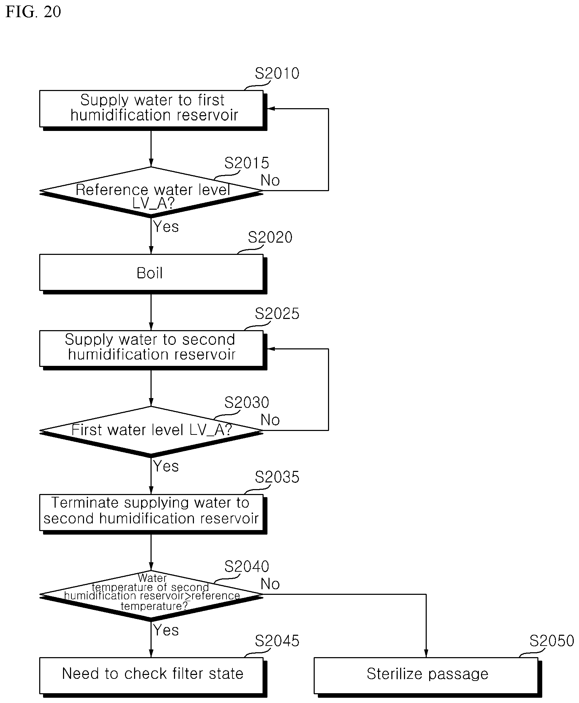

FIG. 20 is a flowchart illustrating a method for operating a humidifier according to an embodiment of the present invention.

Referring to FIG. 20, the control unit 1540 may supply water to the first humidification reservoir 300 (S2010).

When the reference water level LV_A is sensed by the first water level sensor 338 (2015), the control unit 1540 may operate the heater 340 to boil water (S2020).

The control unit 1540 may open the first connection pipe 390 and supply the water heated by the first humidification reservoir 300 to the second humidification reservoir 350 (S2025).

When the first water level LV_1 is sensed by the second water level sensor 366 (S2030), the control unit 1540 may control the second valve 402 to terminate water supply to the second humidification reservoir 350 (S2035).

Then, the state of the filter 50 may be determined based on the water temperature of the second humidification reservoir 350 sensed by the second temperature sensor 364.

When a state in which the water temperature of the second humidification reservoir 350 is equal to or higher than a check reference temperature is maintained for a check time or longer (S2040), the control unit 1540 may determine this state as a filter state check requirement state (S2045).

In addition, the control unit 1540 may control the output unit 1520 or the communication unit 1570 to output a filter state check requirement notification.

When the state in which the water temperature of the second humidification reservoir 350 is equal to or higher than the check reference temperature is not maintained for the check time or longer (S2040), the control unit 1540 may control the passage sterilization operation to be performed (S2050).

The control unit 1540 may control a sterilization operation of supplying water up to the reference water level LV_A to the first chamber 300a, and operating the heater 340 at the first output, boiling the water within the first chamber 300a, and then switching an output of the heater 340 to a second output, and maintaining the heating state for a predetermined time to be performed a predetermined number of times (S2050).

According to an embodiment, even when the state in which the water temperature of the second humidification reservoir 350 is equal to or higher than the check reference temperature is maintained for the check time or longer (S2040), the control unit 1540 may control the passage sterilization operation to be performed (S2050). Even in this case, the control unit 1540 may control the output unit 1520 or the communication unit 1570 to output the filter state check requirement notification to provide information to the user.

Throughout the document, preferred embodiments of the present invention have been described with reference to appended drawings; however, the present invention is not limited to the embodiments above. Rather, it should be noted that various modifications of the present invention may be made by those skilled in the art to which the present invention belongs without leaving the technical scope of the present invention defined by the appended claims, and these modifications should not be understood individually from the technical principles or perspectives of the present invention.

## Claims

1. A humidifier comprising:
a water tank (100) forming a space in which water is held;
a first humidification reservoir (300) having a first chamber (300a) and connected to the water tank (100) through a supply tube (230);
a first water level sensor (338) configured to sense a water level of water held within the first humidification reservoir (300);
a heater (340) configured to heat water in the first chamber (300a);
a first valve (400) configured to open and close the supply tube (230);
a second humidification reservoir (350) having a second chamber (350a) and connected to the first humidification reservoir (300) through a communication pipe (310); and
a first discharge passage (32) through which humidified air discharged from the second humidification reservoir (350) moves,
wherein in a passage sterilization mode, water is supplied to the first chamber (300a) up to a reference water level, and the heater (340) is operated at a first output to boil the water within the first chamber (300a).

2. The humidifier of claim 1, wherein an output of the heater (340) is switched to a second output lower than the first output, and a heating state is maintained for a predetermined time.

3. The humidifier of claim 2, wherein a sterilization operation is repeatedly performed, which supplies water to the first chamber (300a) up to the reference water level, operates the heater (340) at the first output, and boil the water within the first chamber (300a), and then switches the output of the heater (340) to the second output, and maintains the heating state for the predetermined time.

4. The humidifier of any one of claims 1 to 3, further comprising:
a first temperature sensor (336) configured to sense a temperature of the water held within the first humidification reservoir (300),
wherein when a water temperature of the first humidification reservoir (300) reaches 100 degrees Celsius or reaches a first temperature lower than 100 degrees Celsius, and then is maintained for a reference time, this state is determined as a boiling state.

5. The humidifier of any one of claims 2 to 4, further comprising:
a first connection pipe (390) connecting the first humidification reservoir (300) and the second humidification reservoir (350), in which the water heated by the first humidification reservoir (300) moves; and
a second valve (402) configured to open and close the first connection pipe (390).

6. The humidifier of claim 5, wherein in the passage sterilization mode, water is supplied to the first chamber (300a) up to the reference water level, and the heater (340) is operated at the first output to boil the water within the first chamber (300a), and then the first connection pipe (390) is opened.

7. The humidifier of claim 5 or 6, further comprising:
a second water level sensor (366) configured to sense a water level of water held within the second humidification reservoir (350),
wherein when a first water level is sensed by the second water level sensor (366), the first connection pipe (390) is closed.

8. The humidifier of any one of claims 1 to 7, further comprising:
a second temperature sensor (364) configured to sense the temperature of the water held within the second humidification reservoir (350), and
a filter (50) placed below the second humidification reservoir (350).

9. The humidifier of claim 8, wherein after closing the first connection pipe (390), when a state in which the water temperature of the second humidification reservoir (350) is equal to or higher than a preset check reference temperature is maintained for a check time or longer, a filter state check requirement notification is output.

10. The humidifier of claim 8 or 9, further comprising:
a blower fan (62) placed between the first humidification reservoir (300) and the filter /50), and forming air movement,
wherein the blower fan (62) is configured to rotate in response to an operation of the heater (340).

11. The humidifier of any one of claims 8 to 10, further comprising:
a second discharge passage (34) through which air filtered by the filter (50) moves; and
a mixture passage (13) in which the air moving through the first discharge passage (32) and the air moving through the second discharge passage (34) are mixed.

12. The humidifier of claim 11, wherein an air volume of the air moving through the second discharge passage (34) is larger than an air volume of the air moving through the first discharge passage (32).

13. The humidifier of any one of claims 10 to 12, wherein in the second humidification reservoir (350), an exhaust hole through which humidified air generated by the second chamber (350a) is discharged to the outside and an air supply hole through which air moving by the blower fan (62) is supplied to the second chamber (350a).

14. The humidifier of any one of claims 1 to 13, wherein the passage sterilization mode is preferentially performed before a humidification operation is performed.

15. The humidifier of claim 14, wherein water is supplied until the water level of the first humidification reservoir (300) reaches the reference water level, and
the passage sterilization mode is switched to the humidification operation.
